# EUROPEAN PATENT APPLICATION

(11) **EP 4 813 297 A2**
(43) Date of publication of application: **30.09.2026**
(21) Application number: 26198158.3
(22) Date of filing: 31.05.2023
(51) Int. Cl.: A61B 5/369

(54) **HEARING SYSTEM WITH HEARING DEVICE BASED HEALTH CHARACTERIZATION AND/OR MONITORING AND RELATED METHODS**

(30) Priority: 31.05.2022 DK PA202270286
(62) Document number(s) of the earlier application(s) in accordance with Art. 76 EPC: 23729766.8 / 4 531 660
(71) Applicant: GN Hearing A/S, 2750 Ballerup (DK)
(72) Inventor: BRANDT, Sigurd, 2750 Ballerup (DK); COSTA, Alex Ignatius, 2750 Ballerup (DK); VILLADSEN, Peter Aksel, 2750 Ballerup (DK)
(74) Representative: Zacco Denmark A/S

(57) **Abstract**

A hearing system is disclosed. The hearing system comprises an electronic device comprising a memory, an interface, and a processor. The hearing system comprises a hearing device comprising a memory, an interface, a processor, and one or more sensors. The system is configured to obtain sensor data, such as external sensor data from the one or more sensors. The system is configured to determine, based on the sensor data, such as the external sensor data, a health parameter indicative of a cognitive state of a user of the hearing device. The system is configured to determine whether the health parameter satisfies a first criterion indicative of a cognitive decline. The system is configured, in accordance with the health parameter satisfying the first criterion, to output a health representation associated with the cognitive state.

## Description

The present disclosure pertains to the field of hearing systems, and in particular to hearing systems with hearing device based health characterization and/or monitoring and related methods.

### BACKGROUND

Hearing loss has been associated with cognitive impairment and dementia and identified as the biggest modifiable risk factors for dementia if addressed early in midlife, e.g., in the age group of 45-65 years old. Additionally, according to a Lancet commission, the risk for development towards dementia rose for each additional 10 decibel loss in hearing.

Furthermore, for people identified with mild cognitive impairment, it has been shown that using hearing aids can lead to a significantly reduced risk of developing dementia compared to people with hearing loss who weren't using hearing aids. The hypothesis among the scientific community is that hearing loss leads to an increased cognitive load which leads to recruitment of other parts of the brain at the detriment of other functions and over time leads to reduced cognitive reserve until dementia sets in. The WHO estimates that 55 million people worldwide suffer from dementia, the majority of which live in low and middle-income countries. The global prevalence is anticipated by WHO to rise to 139 million by 2050. Alzheimer's and associated dementias are the seventh leading cause of death and one of the major issues affecting the quality of life for elder people.

### SUMMARY

There is currently a lack of long term, accurate and convenient technologies for characterizing and/or monitoring health of users, such as characterizing and/or monitoring the cognitive state of a user and developments thereof.

Accordingly, there is a need for systems for health characterization and/or monitoring and methods for health characterization and/or monitoring, which may mitigate, alleviate, or address the shortcomings existing and may provide improved health characterization and/or monitoring of a user with improved cognitive state characterization and/or monitoring and with increased awareness regarding cognitive health.

A hearing device is disclosed. The hearing device may be seen as a hearing device with health characterization and/or monitoring. The hearing device comprises a memory, an interface, a processor, and one or more sensors, wherein the processor is configured to obtain, such as receive and/or retrieve, sensor data from the one or more sensors; determine, based on the sensor data, a health parameter, e.g. indicative of a cognitive state of a user of the hearing device; and optionally determine whether the health parameter satisfies a first criterion, e.g. indicative of a cognitive decline. Optionally, the processor is configured to, e.g. in accordance with the health parameter satisfying the first criterion, output, e.g. via the interface, a health representation associated with the cognitive state.

A method of operating a hearing device is disclosed. The method comprises obtaining, from one or more sensors of the hearing device, sensor data; determining, based on the sensor data, a health parameter, e.g. indicative of a cognitive state of a user of the hearing device; and determining whether the health parameter satisfies a first criterion, e.g. indicative of a cognitive decline. Optionally, the method comprises, e.g. in accordance with the health parameter satisfying the first criterion, outputting a health representation associated with the cognitive state.

Further, an electronic device is disclosed. The electronic device comprises a memory, an interface, and a processor. The processor is configured to obtain, via the interface, external sensor data from a hearing device; determine, based on the external sensor data, a health parameter, e.g. indicative of a cognitive state of a user of the hearing device. The processor may be configured to determine whether the health parameter satisfies a first criterion indicative of a cognitive decline. Optionally, the processor is configured, e.g. in accordance with the health parameter satisfying the first criterion, to output, e.g. via the interface, a health representation associated with the cognitive state.

Additionally, a method of operating an electronic device is disclosed. The method comprises obtaining sensor data, such as external sensor data from a hearing device; determining, based on the sensor data, such as the external sensor data, a health parameter e.g. indicative of a cognitive state of a user of the hearing device; and determining whether the health parameter satisfies a first criterion e.g. indicative of a cognitive decline. Optionally, the method comprises, e.g. in accordance with the health parameter satisfying the first criterion, outputting a health representation associated with the cognitive state.

Further, a hearing system is disclosed. The hearing system comprises an electronic device comprising a memory, an interface, and a processor. The hearing system comprises a hearing device comprising a memory, an interface, a processor, and one or more sensors. The system is configured to obtain sensor data, such as external sensor data from the one or more sensors. The system is configured to determine, based on the sensor data, such as the external sensor data, a health parameter indicative of a cognitive state of a user of the hearing device. The system is configured to determine whether the health parameter satisfies a first criterion indicative of a cognitive decline. Optionally, the system is configured, in accordance with the health parameter satisfying the first criterion, to output a health representation associated with the cognitive state.

A method of operating a hearing system is disclosed. The method comprises obtaining sensor data, such as external sensor data from a hearing device. The method comprises determining, based on the sensor data, such as the external sensor data, a health parameter indicative of a cognitive state of a user of the hearing device. The method comprises determining whether the health parameter satisfies a first criterion indicative of a cognitive decline. Optionally, the method comprises, in accordance with the health parameter satisfying the first criterion, outputting a health representation associated with the cognitive state.

The disclosed hearing system, hearing device, electronic device, and related methods provide improved health characterization and/or monitoring. In other words, the present disclosure may provide improved sensor-based (such as audio-based) health characterization and/or monitoring of a user of a hearing device, with improved accuracy and precision. For example, a distance between the hearing device and a mouth of the user may be constant and close which may provide sensor data of an improved accuracy. Further, by using a hearing device to provide sensor data, a sensor may be positioned close to an artery, which may provide better measurements and in turn more accurate sensor data. It may be appreciated that by using a hearing device to provide sensor data, fewer movement artifacts may occur, e.g., compared to sensors positioned at extremities of a user.

The present disclosure allows for improved cognitive decline detection by determining a health parameter based on sensor data, such as based on sensor data indicative of a hearing device user's biomarkers, e.g., voice biomarkers, physiological biomarkers, and/or biokinetic biomarkers.

It may be appreciated that the present disclosure may provide improved feedback on a cognitive state of a user, the feedback being more intelligible for the user. The present disclosure provides an improved awareness regarding health of a user, especially regarding cognitive health of a user of a hearing device. In other words, the present disclosure may provide an increased awareness about the importance of cognitive health and diseases related to cognitive decline. For example, by providing the health representation the present disclosure may improve the visualization and/or the intelligibility to a user of an hearing device. The health representation may therefore provide information about an cognitive state e.g., based on a health parameter. Hearing devices are known to be worn for long periods of time during a day, and therefore a hearing device with health characterization and/or monitoring capabilities increases the probability of e.g., cognitive decline detection.

In turn, the present disclosure may provide a customized health representation, e.g., based on long term sensor data, to a user of a hearing device. Furthermore, the present disclosure may encourage an increased usage of hearing devices, e.g., to reduce the risk of cognitive decline.

It may be appreciated that the present disclosure provides health characterization and/or monitoring of users of hearing devices, for example to perform early detection and/or identification of cognitive decline of a user of a hearing device. For example, a user of a hearing device may be continuously screened for cognitive decline, such as cognitive disorder. This may in turn reduce the long term healthcare costs and reduce the burden on caregivers and the healthcare system. The present disclosure provides a flexible and non-invasive way of characterizing and/or monitoring health of a user of a hearing device, e.g., by using sensor data from a hearing device. In other words, the present disclosure may provide a low-cost technology for performing health characterization and/or monitoring of users of hearing devices, which can also be performed remotely at home for the user.

An advantage of the present disclosure is that it is possible to characterize and/or monitor the health of a user of a hearing device based on sensor data obtained over long periods of time, e.g., the user of a hearing device is usually wearing the hearing device during a substantial part of the awake hours.

### BRIEF DESCRIPTION OF THE DRAWINGS

The above and other features and advantages of the present disclosure will become readily apparent to those skilled in the art by the following detailed description of examples thereof with reference to the attached drawings, in which:
Fig. 1 schematically illustrates an example hearing system according to the present disclosure,
Fig. 2 is a flow chart of an example method according to the present disclosure,
Fig. 3 is a flow chart of an example method according to the present disclosure, and
Fig. 4A-B is a flow chart of an example method according to the present disclosure.

### DETAILED DESCRIPTION

Various examples and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the examples. They are not intended as an exhaustive description of the disclosure or as a limitation on the scope of the disclosure. In addition, an illustrated example needs not have all the aspects or advantages shown. An aspect or an advantage described in conjunction with a particular example is not necessarily limited to that example and can be practiced in any other examples even if not so illustrated, or if not so explicitly described.

The figures are schematic and simplified for clarity, and they merely show details which aid understanding the disclosure, while other details have been left out. Throughout, the same reference numerals are used for identical or corresponding parts.

A hearing system is disclosed. The hearing system may be seen as a system for performing health characterizing and/or monitoring. In other words, the hearing system may be seen as a system for performing health characterization and/or monitoring of a user of a hearing device, such as a user wearing a hearing device.

The hearing system comprises an electronic device comprising a memory, an interface, and a processor. The electronic device of the hearing system may be an electronic device as disclosed herein. The hearing system comprises a hearing device comprising a memory, an interface, a processor, and one or more sensors. The hearing device of the hearing system may be a hearing device as disclosed herein.

The hearing device as disclosed herein may comprise one or more processors, a memory, an interface and one or more transducers, such as microphone(s), such as a first microphone and/or a receiver, such as a hearing device speaker.

The hearing device may be configured to be worn at an ear of a user. The hearing device may be a hearable. The hearing device may be a hearing aid, wherein the processor(s) is configured to compensate for a hearing loss of a user.

The hearing device may be an ear bud, a hearing aid, a headset, a personal sound amplification product (PSAP), an over-the-counter (OTC) hearing device, a hearing protection device, a custom hearing device or another ear-wearable hearing device. Hearing devices can include both prescription devices and non-prescription devices.

The hearing device may be embodied in various housing styles/form factors. Some of these form factors are a Behind-the-Ear (BTE) hearing device, a Receiver-in-Canal (RIC) hearing device, a Receiver-in-Ear (RIE) hearing device, or a Microphone-and-Receiver-in-Ear (MaRIE) hearing device. These devices have in common that they may comprise a BTE component configured to be worn behind the ear of the user and an in the ear (ITE) component configured to be inserted partly or fully into the user's ear canal. Generally, the BTE component may comprise at least one input transducer, a power source, and a processing unit. The term BTE hearing device may refer to a hearing device where the receiver, i.e. the output transducer, is comprised in the BTE component and sound is guided to the ITE component via a sound tube connecting the BTE and ITE components, whereas the terms RIE, RIC, and MaRIE devices refers to hearing devices where the receiver is comprised in the ITE component, which is coupled to the BTE component via a connector cable or wire configured for transferring electric signals between the BTE and ITE components.

The hearing device may be of the behind-the-ear (BTE) type, in-the-ear (ITE) type, in-the-canal (ITC) type, receiver-in-canal (RIC) type or receiver-in-the-ear (RITE) type. The hearing aid may be a binaural hearing aid.

In one or more example hearing systems, hearing devices, and/or electronic devices, the hearing device may be seen as a user hearing device, such as a headphone, an earphone, a hearing aid, an over-the-counter (OTC) hearing device, and/or a hearing protection device. In one or more example hearing systems and/or hearing devices, the hearing device may comprise one or more transceivers for wireless communication. In one or more example hearing systems and/or hearing devices, the hearing device may facilitate wired communication, such as by using cable, such as an electrical cable.

The hearing device may be configured for wireless communication, e.g. via the interface, with one or more devices, such as with another hearing device, e.g. as part of a binaural hearing system, and/or with one or more accessory devices/electronic devices, such as a smartphone and/or a smart watch. The hearing device optionally comprises an antenna for converting one or more wireless input signals. The wireless input signal(s) may origin from external source(s), such as spouse microphone device(s), wireless TV audio transmitter, and/or a distributed microphone array associated with a wireless transmitter. The wireless input signal(s) may origin from another hearing device, e.g. as part of a binaural hearing system, and/or from one or more accessory devices. The sensor data as disclosed herein may be based on one or more wireless input signals, e.g., from an external source. This may for example provide information regarding a user's environment, information which may be used to determine a health parameter as disclosed herein.

The hearing device optionally comprises a radio transceiver coupled to the antenna for converting the antenna output signal to a transceiver input signal. Wireless signals from different external sources may be multiplexed in the radio transceiver to a transceiver input signal or provided as separate transceiver input signals on separate transceiver output terminals of the radio transceiver. The hearing device may comprise a plurality of antennas and/or an antenna may be configured to be operate in one or a plurality of antenna modes.

In one or more hearing systems, hearing devices, and/or electronic devices, the one or more sensors comprise a microphone for provision of microphone input data. In other words, the one or more sensors may comprise a set of microphones. In one or more hearing systems and/or electronic devices, the external sensor data comprises microphone input data. The microphone may comprise one or more microphones. The microphone may comprise a first microphone for provision of a first microphone input signal and/or a second microphone for provision of a second microphone input signal. The microphone may comprise N microphones for provision of N microphone signals, wherein N is an integer in the range from 1 to 10. In one or more example hearing devices, the number N of microphones is two, three, four, five or more. The microphone may comprise a third microphone for provision of a third microphone input signal. In one or more example hearing devices, the microphone input data may be from a single microphone signal, such as first microphone signal, or a combination of microphone input signals from a plurality of microphones. For example, the microphone data may be based on a first microphone input signal from a first microphone and/or a second microphone input signal from a second microphone. The microphone may comprise an ear canal microphone and/or an ambient microphone for own voice pickup, such as the user's own voice. This may allow to filter out external voices and/or sounds of the environment of the user, e.g., to substantially only pick-up the voice of the user. In one or more hearing systems, hearing devices, and/or electronic devices, the health parameter is based on the microphone input data.

In one or more hearing systems, hearing devices, and/or electronic devices, the one or more sensors comprise one or more of: a microphone, an optical sensor, a magnetic sensor, a galvanic sensor, a motion sensor, and a capacitive sensor. An example of an optical sensor is a photoplethysmogram (PPG) sensor. An example of a galvanic sensor is an electrocardiogram (ECG) sensor and/or an electroencephalogram (EEG) sensor. A motion sensor may be seen as an inertial measurement unit (IMU). An example of a motion sensor may comprise an accelerometer and/or a gyroscope. A motion sensor may for example comprise a magnetic sensor, such as a magnetometer. An example of a capacitive sensor may comprise a capacitive microphone and/or a temperature sensor.

In one or more hearing systems, hearing devices, and/or electronic devices, the one or more sensors comprise one or more of a body sensor, e.g., a heart rate sensor, a blood pressure sensor, a pulse sensor, a photoplethysmogram (PPG) sensor, an electrocardiogram (ECG) sensor, an electroencephalogram (EEG) sensor, a bioimpedance sensor, and/or a temperature sensor. The one or more sensors of the hearing device may comprise a body sensor being a capacitive and/or conductivity sensor, e.g. to measure and/or determine stress levels.

The sensor data as disclosed herein may be obtained from one or more of the above sensors. In other words, the sensor data may be generated and/or provided by any one or more of the above mentioned sensors.

The hearing device comprises a processor or a plurality of processors for processing input signals, such as transceiver input signal(s) and/or microphone input signal(s). The processor(s) is optionally configured to compensate for hearing loss of a user of the hearing device. The processor(s) provides an electrical output signal based on the input signals to the processor. Input terminal(s) of the processor are optionally connected to respective microphones and/or output terminals of a pre-processing unit. One or more microphone input terminals of the processor may be connected to respective one or more microphone output terminals of the pre-processing unit.

The hearing system, the hearing device, and/or the electronic device may be configured for wireless communications via a wireless communication system, such as short-range wireless communications systems, such as Wi-Fi, Bluetooth, Zigbee, IEEE 802.11, IEEE 802.15, infrared and/or the like.

The hearing system, the hearing device and/or the electronic device (such as accessory device) may be configured for wireless communications via a wireless communication system, such as a 3GPP system, such as a 3GPP system supporting one or more of: New Radio, NR, Narrow-band IoT, NB-IoT, and Long Term Evolution - enhanced Machine Type Communication, LTE-M, millimeter-wave communications, such as millimeter-wave communications in licensed bands, such as device-to-device millimeter-wave communications in licensed bands.

In one or more hearing systems, hearing devices, and/or electronic devices, the interface of the electronic device and/or the interface of the hearing device comprises one or more of: a Bluetooth interface, Bluetooth low energy interface, and a magnetic induction interface. For example, the interface of the electronic device and/or the interface of the hearing device may comprise a Bluetooth antenna and/or a magnetic interference antenna.

The system is configured to obtain external sensor data from the one or more sensors. In other words, the system is configured to obtain the external sensor data at the hearing device and/or at the electronic device. In other words, the external sensor data may be obtained, received, and/or retrieved by the electronic device from the hearing device. The external sensor data may be transmitted and/or sent by the hearing device to the electronic device. The system is configured to determine, such as using the processor of the hearing device and/or the processor of the electronic device, based on the external sensor data, a health parameter indicative of a cognitive state of a user of the hearing device. In other words, the system is configured to determine, such as at the processor of the hearing device and/or at the processor of the electronic device, based on the external sensor data, a health parameter indicative of a cognitive state of a user of the hearing device. The system is configured to determine, such as using the processor of the hearing device and/or the processor of the electronic device, whether the health parameter satisfies a first criterion indicative of a cognitive decline. In other words, the system is configured to determine, such as at the processor of the hearing device and/or at the processor of the electronic device, whether the health parameter satisfies a first criterion indicative of a cognitive decline. Optionally, the system is configured, in accordance with the health parameter satisfying the first criterion, to output, such as using the processor of the hearing device and/or the processor of the electronic device, via the interface of the hearing device and/or the interface of the electronic device, a health representation associated with the cognitive state.

A hearing device is disclosed. The hearing device may be seen as a hearing device with health characterization and/or monitoring. The hearing device comprises a memory, an interface, a processor, and one or more sensors. The processor is configured to obtain sensor data from the one or more sensors. The processor is configured to determine, based on the sensor data, a health parameter indicative of a cognitive state of a user of the hearing device. The processor is configured to determine whether the health parameter satisfies a first criterion indicative of a cognitive decline. Optionally, the processor is configured, in accordance with the health parameter satisfying the first criterion, to output, via the interface, a health representation associated with the cognitive state.

An electronic device is disclosed. The electronic device comprises a memory, an interface, and a processor. The processor is configured to obtain, via the interface, external sensor data from a hearing device. The processor is configured to determine, based on the external sensor data, a health parameter indicative of a cognitive state of a user of the hearing device. The processor is configured to determine whether the health parameter satisfies a first criterion indicative of a cognitive decline. Optionally, the processor is configured, in accordance with the health parameter satisfying the first criterion, to output, via the interface, a health representation associated with the cognitive state.

The external sensor data and the sensor data as disclosed herein may be used interchangeably. The external sensor data may be seen as sensor data obtained from the hearing device. In other words, the external sensor data may be seen as sensor data generated by the one or more sensors of the hearing device.

In one or more hearing systems, hearing devices, and/or electronic devices, the electronic device comprises one or more sensors, such as one or more internal sensors. In one or more hearing systems, hearing devices, and/or electronic devices, the system is configured to obtain internal sensor data from the one or more sensors of the electronic device. In one or more hearing systems, hearing devices, and/or electronic devices, the health parameter is based on the internal sensor data. The internal sensor data may be seen as sensor data obtained from the electronic device. In other words, the internal sensor data may be seen as sensor data generated by the one or more sensors of the electronic device. It may be appreciated that the health parameter is determined based on the sensor data (such as external sensor data) and/or the internal sensor data.

The one or more sensors of the electronic device (such as accessory device) may comprise one or more of: an optical sensor, a magnetic sensor, a galvanic sensor, a motion sensor, and a capacitive sensor. An example of an optical sensor is a photoplethysmogram (PPG) sensor. An example of a galvanic sensor is an electrocardiogram (ECG) sensor and/or an electroencephalogram (EEG) sensor. A motion sensor may be seen as an inertial measurement unit (IMU). An example of a motion sensor may comprise an accelerometer and/or a gyroscope. A motion sensor may for example comprise a magnetic sensor, such as a magnetometer. An example of a capacitive sensor may comprise a capacitive microphone and/or a temperature sensor.

In one or more hearing systems, hearing devices, and/or electronic devices, the one or more sensors of the electronic device (such as the accessory device) comprise one or more of a body sensor, e.g., a heart rate sensor, a blood pressure sensor, a pulse ox sensor, a photoplethysmogram (PPG) sensor, an electrocardiogram (ECG) sensor, an electroencephalogram (EEG) sensor, a bioimpedance sensor, and a temperature sensor. The one or more sensors of the hearing device may comprise a body sensor being a capacitive and/or conductivity sensor, e.g. to measure and/or determine stress levels.

The internal sensor data as disclosed herein may be obtained from one or more of the above sensors. In other words, the internal sensor data may be generated and/or provided by any one or more of the above mentioned sensors.

In one or more example hearing systems, hearing devices, and/or electronic devices, the one or more sensors comprise one or more motion sensors, e.g., an accelerometer, an inertial motion sensor, a gyroscope, an altimeter, and/or a position sensor such as a GPS sensor.

In one or more example hearing systems, hearing devices, and/or electronic devices, the body sensors are configured to determine body data indicative of user's body information for provision of the health parameter. In one or more example hearing systems, hearing devices, and/or electronic devices, the motion sensors are configured to determine motion data indicative of user's motion information for provision of the health parameter.

In one or more example hearing systems, hearing devices, and/or electronic devices, a user may be seen a user of the hearing device and/or a user of the electronic device (such as a user of the accessory device).

In one or more example hearing systems and/or electronic devices, the electronic device is a server device.

In one or more example hearing system and/or electronic devices, the electronic device is an accessory device.

The electronic device may comprise an accessory device and/or a server device. The electronic device may be configured to operate on an accessory device and/or a server device. In other words, the electronic device may be configured to act as a server device and/or an accessory device. An accessory device may for example be or comprise a mobile phone, such as a smartphone, a smart-watch, smart-speakers, a tablet, a computer, such as a laptop computer or PC, or a tablet computer. In other words, the electronic device may for example be a user device, such as a mobile phone or a computer, configured to perform a health characterization and/or monitoring. In one or more example hearing systems and/or electronic devices, the accessory device may be seen as a user accessory device, such as a mobile phone, a smart watch, a tablet, and/or a wearable gadget. In one or more example hearing systems, the accessory device may comprise one or more transceivers for wireless communication. In one or more example hearing systems, the accessory device may facilitate wired communication, such as by using a cable, such as an electrical cable.

A server device may be configured on a cloud, such as a cloud network. Different operations configured to be performed by the electronic device and/or the system as disclosed herein may be performed at different devices, such as at the electronic device and/or at the server device.

A health parameter as disclosed herein is indicative of a cognitive state of a user of the hearing device. A health parameter may also be seen as and/or denoted a cognitive parameter. In other words, the health parameter may be indicative of information associated with a cognitive state of the user of the hearing device. A cognitive state of a user may be indicative of the user's abilities to hear, to speak, to see, and/or to move. In other words, the health parameter may be indicative of the user's abilities to hear, to speak, to see, and/or to move. For example, a cognitive parameter may be seen as a suite of features which together will constitute a biomarker. For example, based on the microphone input data, a cognitive parameter may be seen as a suite of audio features which together will constitute a voice biomarker. For example, based on the physiological data, a cognitive parameter may be seen as one or more physiological features which together will constitute a physiological biomarker. For example, based on the biokinetic data, a cognitive parameter may be seen as one or more biokinetic features which together will constitute a biokinetic biomarker. A health parameter may be indicative of a cognitive load of the user. A health parameter may be determined based on one or more biomarker as disclosed herein, such as based on one or more of voice biomarkers, physiological biomarker, and/or biokinetic biomarkers. For example, the cognitive load may be determined based on one or more biomarker as disclosed herein, such as based on one or more of voice biomarkers, physiological biomarker, and/or biokinetic biomarkers.

It may be appreciated that the health parameter may comprise a score, such as a health score, indicative of a cognitive state of the user of the hearing device. In other words, to determine a health parameter may comprise to determine a health score indicative of a cognitive state of the user. To determine whether the health parameter satisfies a first criterion indicative of a cognitive decline may comprise to determine whether the health score satisfies the first criterion. For example, when the score is above or equal to a threshold (such as the first threshold), it may be determined that the score satisfies the criterion (such as first criterion). A cognitive state may be indicative of a degree of cognitive abilities. A cognitive ability may comprise a memory ability, a stress ability (the ability to work under load), a language ability, a thinking ability, and/or a judgement ability. A cognitive state may for example be indicative of a mild cognitive impairment. Mental diseases such as anxiety and depression may have an influence on the cognitive abilities of a user. A cognitive state may therefore be indicative of a mental disease.

In one or more example hearing systems, in accordance with the health parameter not satisfying the first criterion, the system is configured to refrain from outputting a health representation associated with the cognitive state.

In one or more example hearing devices, in accordance with the health parameter not satisfying the first criterion, the processor of the hearing device is configured to refrain from outputting a health representation associated with the cognitive state.

In one or more example electronic devices, in accordance with the health parameter not satisfying the first criterion, the processor of the electronic device is configured to refrain from outputting a health representation associated with the cognitive state.

The first criterion may comprise a threshold, such as a health threshold and/or cognitive threshold, indicative of cognitive decline of the user of the hearing device. The first criterion may be based on one or more user parameters. For example, the first criterion may be based on an age of the user. The first criterion may therefore be indicative of a cognitive decline with respect to the one or more user parameters. For example, the first criterion may be indicative of a cognitive decline with respect to the age of the user, e.g., in comparison to the normal cognitive capabilities of a person of the same age. The one or more user parameters may also comprise historical user data of the user of the hearing device. The first criterion may therefore be indicative of a cognitive decline of the user over time based on historical data. The first criterion may be based on historical health parameters of the user of the hearing device. Thereby, it may be possible to determine whether the user suffers of cognitive decline over time, with respect to the user's own historical health parameters, such as cognitive capabilities.

To determine whether the health parameter satisfies a first criterion may comprise to determine whether the health parameter is above, below, or equal to a health threshold and/or is within a certain range indicative of the first criterion. In other words, the health parameter may satisfy the first criterion when the health parameter is above or equal to a first threshold. For example, the health parameter may satisfy the first criterion when the health parameter is above or equal to a first threshold indicative of cognitive decline. In other words, when the health parameter satisfies the first criterion, the user of the hearing device may be seen as having a cognitive decline. Formulated differently, when the health parameter satisfies the first criterion one or more cognitive abilities of the user of the hearing device are determined to be declining. For example, the user's abilities to hear, to speak, to see, and/or to move may be determined to be declining.

A cognitive decline of a health parameter, such as of a cognitive state of a user, may be determined based on the user's prior, such as historical, cognitive state, and/or based on a normal cognitive state of a person. In other words, a cognitive decline of a cognitive state of a user may be determined in comparison with the user's historical cognitive state and/or in comparison with on a normal cognitive state of a person of approximately the same age, the same gender, and/or having approximately the same physiological state. Cognitive decline may be seen as a deterioration of cognitive abilities such as memory, attention, problem-solving, speech abilities, and/or language skills. In other words, cognitive decline may be seen as a deterioration of cognitive abilities over time, e.g., a deterioration of cognitive abilities over time in comparison with the user's historical cognitive state and/or in comparison with on a normal cognitive state of a person of approximately the same age, the same gender, and/or having approximately the same physiological state.

In one or more example hearing systems, the system is configured to determine, such as at the electronic device and/or at the hearing device, the health representation associated with the cognitive state based on the sensor data, such as based on the external sensor data. In other words, the system is configured to determine, such as using the processor of the electronic device and/or the processor of the hearing device, the health representation associated with the cognitive state based on the sensor data, such as based on the external sensor data.

In one or more example electronic devices, the processor is configured to determine the health representation associated with the cognitive state based on the sensor data, such as based on the external sensor data.

In one or more example hearing devices, the processor is configured to determine the health representation associated with the cognitive state based on the sensor data.

In one or more example hearing systems, the system comprises machine learning circuitry configured to operate according to a machine learning model. In one or more example hearing systems, to determine a health parameter comprises to determine, such as using the electronic device and/or the hearing device, the health parameter, based on the external sensor data, using the machine learning model. In other words, the electronic device and/or the hearing device may comprise machine learning circuitry configured to operate according to a machine learning model. In other words, the system may be configured to determine, such as using the electronic device and/or the hearing device, the health parameter, based on the external sensor data, using the machine learning model.

In one or more example hearing devices, the processor comprises machine learning circuitry configured to operate according to a machine learning model. In one or more example hearing devices, to determine a health parameter comprises to determine the health parameter, based on the sensor data, using the machine learning model.

In one or more example electronic devices, the processor comprises machine learning circuitry configured to operate according to a machine learning model. In one or more example electronic devices, to determine a health parameter comprises to determine the health parameter, based on the external sensor data, using the machine learning model.

In other words, the electronic device and/or the hearing device may comprise one or more processors comprising a machine learning engine configured to operate according to the machine learning model. The machine learning circuitry may be seen as a predictor circuitry configured to operate according to a prediction model. The machine learning circuitry may be configured to predict, based on the sensor data (such as external sensor data), a health parameter using the prediction model.

The machine learning model may for example comprise or make use of a neural network, artificial intelligence, deep learning, and/or machine learning.

In one or more example hearing systems, electronic devices, and/or hearing devices, the machine learning model comprises model layers including an input layer, one or more intermediate layers, and an output layer for provision of the health parameter. In one or more example hearing systems, electronic devices, and/or hearing devices, the machine learning model comprises a neural network. In one or more example hearing systems, electronic devices, and/or hearing devices, the machine learning model comprises neural network layers including an input layer, one or more intermediate layers, and an output layer for provision of the health parameter. In other words, the input layer, the one or more intermediate layers, and/or the output layer may be seen as layers of a machine learning model such as layers of a neural network. The one or more intermediate layers may be considered as hidden layers (such as hidden features). The one or more intermediate layers may include a first intermediate layer. The machine learning model may comprise a look-up table with a plurality of parameters, such as at least four parameters.

A model as referred to herein (such as the machine learning model) may be seen as a model and/or a scheme and/or a mechanism and/or a method configured to provide, based on operational data (such as sensor data, e.g., audio input data) and/or a previous model, one or more health parameters. A model as referred to herein (such as the machine learning model) may be based on the same model architecture. A model architecture may be based on a neural network, such as comprising one or more different type of layers and/or number of layers. A model architecture may be seen as configuration of a model, such as comprising one or more parameters of a model.

In one or more example hearing systems, electronic devices, and/or hearing devices, the model as referred to herein may be stored on a non-transitory storage medium (for example, on the memory of the electronic device and/or the hearing device). The model may be stored on a non-transitory storage medium of the electronic device and/or the hearing device being configured to execute the model. In one or more example hearing systems, electronic devices, and/or hearing devices, the model may comprise model data and or computer readable instructions (for example based on sensor data and/or audio signal, such as historical sensor data). The model data and/or the computer readable instructions may be used by the electronic device and/or the hearing device. The model (such as model data and/or the computer readable instructions) may be used by the hearing device and/or the electronic device to determine health parameters and health representations. In other words, the model (such as model data and/or the computer readable instructions) may be used by the hearing device and/or the electronic device to determine one or more parameters, features, and/or biomarkers as described herein, such as health parameters, voice biomarkers, physiological biomarkers, and/or biokinetic biomarkers. Generally, the biomarkers as disclosed herein, such as the voice biomarkers, the physiological biomarker, and/or the biokinetic biomarkers, may be indicative of and/or representative of an energy usage and/or level of the user. The energy usage and/or level may provide indications regarding the cognitive state, such as the cognitive load, of the user.

In one or more example electronic devices, the machine learning circuitry comprises a neural network module configured to operate according to a neural network.

In one or more example hearing systems, electronic devices, and/or hearing devices, the neural network is a deep neural network, such as a convolutional neural network and/or a recurrent neural network. For example, the neural network may comprise a one dimensional convolutional neural network or a two dimensional convolutional neural network.

In one or more example hearing systems, electronic devices, and/or hearing devices, the machine learning circuitry comprises a regressor module configured to operate according to a regression model.

The machine learning model may be based on a neural network (such as a convolutional neural network, a deep learning neural network, a recurrent neural network, and/or a combined learning circuitry). The machine learning circuitry may be configured to determine (and optionally identify) one or more patterns in existing data (sensor data, audio input data, sound patterns (such as voice patterns), and/or health parameters) in order to facilitate making determinations and/or predictions for subsequent health parameters. For example, the machine learning circuitry may be configured to determine (such as recognize) a health parameter based on sensor data and/or audio input data over time.

The machine learning circuitry (such as the neural network module and/or the regressor module) may be configured to operate according to a machine learning scheme configured to determine a rule or a pattern or a relation that maps inputs to outputs, so that when subsequent novel inputs are provided the machine learning circuitry may, based upon the rule, pattern or relation, accurately predict the correct output. In one or more embodiments, the machine learning model may first extract one or more features from input sensor data, such as by using signal processing methods (such as filters), statistics of the signals (such as mean, max, median, and/or quantile), and/or results from unsupervised learning methods (such as dimension reduction methods, clustering, and/or auto-encoder). The one or more features may then be fed into a regression and/or classification model that is trained using machine learning techniques.

In one or more example hearing systems, electronic devices, and/or hearing devices, the system is configured to train the machine learning model, to provide an updated machine learning model, and to transmit, to the electronic device and/or the hearing device, the updated machine learning model. In other words, the server device is configured to train the machine learning model, to provide an updated machine learning model, and to transmit, to the electronic device and/or the hearing device, the updated machine learning model.

In one or more example hearing systems, electronic devices, and/or hearing devices, the server device is configured to train and/or update the machine learning model based on one or more of: the sensor data and the health parameter. In one or more embodiments, the processor may be configured to train and/or update the machine learning model based on the outcome of the health representation (for example, by comparing the health parameter and known health parameters). The machine learning model that the machine learning circuitry operates according to, may be trained and/or updated (such as retrained or finetuned). The training of the machine learning model may be a supervised learning setup, where the sensor data in the input data can be labelled. The machine learning model or changes to the machine learning model may be based on new data, such as new sensor data, and/or new prediction data.

The health representation as disclosed herein may be seen as a representation indicative of all or part of the health parameter and/or the cognitive state of the user. In other words, the health representation may be seen as an cognitive state representation. The health representation may be seen as and/or comprise an evaluation of a cognitive state of the user. In other words, the health representation may be seen as and/or comprise an evaluation of the cognitive abilities of the user. In one or more example hearing systems, electronic devices, and/or hearing devices, the health representation comprises a score, such as a cognitive score, indicative of a performance of a cognitive state of the user (e.g., when wearing the hearing device). The health representation may indicate whether the cognitive state of the user is indicative of a cognitive decline or not. The health representation may therefore provide feedback regarding the cognitive state of the user, and an indication on whether to act on it or not. For example, the score may be indicative of a cognitive decline when the health parameter satisfies a criterion. For example, the system, the processor of the electronic device, and/or the processor of the hearing device, may be configured to determine whether the score satisfies a criterion (such as the first criterion). When the score is above or equal to a threshold (such as the first threshold), it may be determined that the score satisfies the criterion (such as first criterion). The health representation may comprise information regarding a degree of cognitive decline. For example, the health representation may indicate whether the user suffers of mild cognitive decline, medium cognitive decline, important cognitive decline, or severe cognitive decline. Mild cognitive decline, medium cognitive decline, important cognitive decline, and/or severe cognitive decline may be indicative of and/or denoted as a level of cognitive decline, e.g., from mild cognitive impairment to development of dementia, where mild cognitive decline may be seen as mild cognitive impairment and severe cognitive decline may be seen as development of dementia. A cognitive decline may not necessarily be linked to a mental disorder or condition, but may for example be due to one or more factors such as: tiredness, aging, overstimulation, lifestyle factors, medications, and stress. A cognitive decline may be temporary and may for example be triggered by one or more of the above factors. For example, a deterioration of the cognitive abilities of a user may be determined or detected for a certain period of time but the cognitive abilities of the user may revert to the status before the detected deterioration after a period of time. The determination of a cognitive decline may be different from the determination of a cognitive load or the determination of a mental disorder.

In one or more example hearing systems, electronic devices, and/or hearing devices, the health representation comprises a representation of the health parameter. For example, the health representation may comprise a representation of a biomarker as described herein. The health representation may comprise one or more of: a representation of a voice biomarker representation, a physiological biomarker representation, and a biokinetic biomarker representation.

In one or more example hearing systems, electronic devices, and/or hearing devices, the health representation is indicative of an evaluation of the cognitive state of the user.

In one or more example hearing systems, electronic devices, and/or hearing devices, outputting the health representation may comprise outputting, via the interface of the electronic device and/or the interface of the hearing device, the health representation.

In one or more example hearing systems and/or electronic devices, to output the health representation comprises to display, via the interface of the electronic device, a user interface representing the health representation. In other words, to output the health representation comprises to display, via the interface of the accessory device as disclosed herein, a user interface representing the health representation.

Outputting the health representation may comprise displaying a user interface indicative of the health representation. In one or more example hearing systems and/or electronic devices, outputting the health representation may comprise outputting, via the interface of the electronic device, a first health representation, a second health representation, a third health representation, etc.

Outputting the health representation may comprise displaying a user interface indicative of the health representation.

A user interface may comprise one or more, such as a plurality of, user interface objects. For example, the user interface may comprise one or more user interface objects, such as a first user interface object and/or a second user interface object. A user interface object may refer herein to a graphical representation of an object that is displayed on an interface of the electronic device, such as a display. The user interface object may be user-interactive, or selectable by a user input. For example, an image (e.g., icon), a button, and text (e.g., hyperlink) each optionally constituting a user interface object. The user interface object may form part of a widget. A widget may be seen as a mini-application that may be used by the user. To output the health representation may comprise to output an health representation comprising one or more of text (such as a text string) and/or a phrase, a score (such as an evaluation score and/or a cognitive score), image data (such as one or more images), a sound, an audio message, and/or a user interface object comprising one or more of the previous. For example, to output the health representation may comprise to output a health representation comprising a report of the cognitive state of the user. For example, to output the health representation may comprise to output a health representation comprising a score, such as an evaluation score of the cognitive state of the user with the hearing device.

In one or more example hearing systems, electronic devices, and/or hearing devices, in accordance with the health parameter satisfying the first criterion, the system, the processor of the electronic device, and/or the processor of the hearing device are configured to determine a first recommendation. The first recommendation may be seen as a feedback to the user of the hearing device regarding a cognitive state of the user. The first recommendation may be seen as a first evaluation. The first recommendation may be indicative of a recommendation for avoiding and/or reducing cognitive decline. The first recommendation may be seen as and/or comprise an advisory action that the user of the hearing device should do and/or something that the user should avoid (such as stop doing) in accordance with the health parameter satisfying the first criterion. The first recommendation may for example comprise text (such as a message to the user) and/or phrases such as: "A cognitive decline has been identified, please take contact to a healthcare person and/or to your doctor (such as general practitioner)", "Please perform a cognitive test", "Please increase the usage of your hearing device in order to reduce your cognitive decline", "Please take your hearing device to your audiologist to adjust and/or update your hearing device configuration to reduce your cognitive decline", "Please increase your brain activity and/or your motion activity to reduce your cognitive decline", "Please check for diabetes", "Hypertension has been identified, please take contact to a healthcare person and/or to your doctor (such as general practitioner)", "If you smoke, it is recommended that you quit smoking or at least reduce smoking", "Please try to increase the air quality of your environment, such as reduce the air pollution that you are exposed to", "Please try to lose some weight to reduce your obesity", "Please exercise frequently and/or increase your physical activity level", "If you have a depression, please treat it", "Please avoid excessive alcohol consumption", and/or, "Please try to maintain frequent social contact".

In one or more example hearing systems, electronic devices, and/or hearing devices, the system, the processor of the electronic device, and/or the processor of the hearing device are configured to output, via the interface (such as the interface of the electronic device and/or the hearing device), the first recommendation. In other words, the processor may be configured to output the first recommendation in the form of a text (such as a message to a user) and/or a phrase, an evaluation score, image data (such as one or more images), an audio message (e.g., outputted to the user via a receiver of the hearing device), and/or a user interface as described herein.

In one or more example hearing systems, electronic devices, and/or hearing devices, the first recommendation is comprised in the health representation. In other words, the system, the processor of the electronic device, and/or the processor of the hearing device are configured to include the first recommendation in the health representation. For example, the system, the processor of the electronic device, and/or the processor of the hearing device are configured to output a health representation comprising the first recommendation.

In one or more example hearing systems and/or electronic devices, in accordance with the first criterion being satisfied, the system is configured to perform, via the interface of the electronic device, a cognitive test scheme. In other words, in accordance with the first criterion being satisfied, the system is configured to perform, via the interface of the accessory device, a cognitive test scheme. In one or more example hearing systems and/or electronic devices, in accordance with the first criterion being satisfied, the system is configured to perform, via the interface of the hearing device, a cognitive test scheme.

For example, the system may be configured to output and/or display a user interface representing the cognitive test scheme, e.g., via the interface of the electronic device (e.g., a display) and/or via the interface of the hearing device (e.g., via a receiver of the hearing device). The cognitive test scheme may be configured to be performed, on the electronic device, by the user of the hearing device. To perform the cognitive test scheme may comprise to output, e.g., via the interface of the electronic device (e.g., a display) and/or via the interface of the hearing device (e.g., via a receiver of the hearing device) one or more of: audio data, visual data, text data (such as questionnaire), and to receive one or more inputs from the user via the interface of the electronic device and/or via the interface of the hearing device. The cognitive test scheme may be performed for obtaining more information regarding a cognitive state of the user of the hearing device, e.g., when it has been determined that first criterion is satisfied. The health representation may be based on the health parameter and the user input from the cognitive test scheme. The cognitive test scheme may for example comprise to make the user of the hearing device repeat and/or speak out one or more audio samples, e.g., to test the voice biomarkers of the user when repeating the audio sample(s). The cognitive test scheme may for example test one or more of: a linguistic parameter, an acoustic parameter, a verbal fluency parameter, a mumbling parameter, a voice pitch parameter, and a speech rhythm parameter.

The cognitive test scheme may for example comprise to make the user of the hearing device make one or more movements and/or activities, e.g., to test the physiological biomarkers and/or the biokinetic biomarkers of the user when performing the one or more movements and/or activities. The cognitive test scheme may for example test one or more of: a blood pressure parameter, a blood flow parameter, a heart rate parameter, a respiratory parameter, a temperature parameter, an oxygen parameter, and a brain activity parameter. The cognitive test scheme may for example test one or more of: a motion parameter, a trembling parameter, a shaking parameter, and a tic parameter.

In one or more example hearing systems and/or electronic devices, the cognitive test scheme may be comprised in a health representation and/or the health representation may be based on the cognitive test scheme.

In one or more example hearing systems, electronic devices, and/or hearing devices, to determine a health parameter comprises to determine, based on the microphone input data, a first voice biomarker. To determine a health parameter may for example comprise to determine a second voice biomarker, a third voice biomarker, a fourth voice biomarker, a fifth voice biomarker, and/or a sixth voice biomarker. To determine a voice biomarker, such as the first voice biomarker, may comprise to extract one or more features from the sensor data. To determine a voice biomarker, such as the first voice biomarker, may comprise to extract one or more audio features from microphone input data. A voice biomarker may be seen as a biomarker indicative of one or more parameters of a voice, such as the voice of the user of the hearing device. A voice biomarker may comprise and/or be indicative of a signature, such as a sound signature, in the microphone input data.

In one or more example hearing systems, electronic devices, and/or hearing devices, the first criterion is based on the first voice biomarker. In other words, the first criterion comprises a first voice biomarker criterion. In other words, to determine whether the health parameter satisfies the first criterion may comprise to determine whether the health parameter satisfies the first voice biomarker criterion.

In one or more example hearing systems, electronic devices, and/or hearing devices, to determine the first voice biomarker comprises to determine one or more of: a linguistic parameter, an acoustic parameter, a verbal fluency parameter, a mumbling parameter, a voice pitch parameter, and a speech rhythm parameter. In one or more example hearing systems, the system is configured, such as using the electronic device and/or the hearing device, to determine one or more of: a linguistic parameter, an acoustic parameter, a verbal fluency parameter, a mumbling parameter, a voice pitch parameter, and a speech rhythm parameter.

In one or more example electronic devices, the processor of the electronic device is configured to determine one or more of: a linguistic parameter, an acoustic parameter, a verbal fluency parameter, a mumbling parameter, a voice pitch parameter, and a speech rhythm parameter.

In one or more example hearing devices, the processor of the hearing device is configured to determine one or more of: a linguistic parameter, an acoustic parameter, a verbal fluency parameter, a mumbling parameter, a voice pitch parameter, and a speech rhythm parameter.

In one or more example hearing systems, electronic devices, and/or hearing devices, the first voice biomarker is based on one or more of: a linguistic parameter, an acoustic parameter, a verbal fluency parameter, a mumbling parameter, a voice pitch parameter, and a speech rhythm parameter.

A linguistic parameter may be seen as a parameter indicative of a linguistic biomarker. In other words, a linguistic parameter may comprise specific semantic related words and/or sentences, such as incomplete words and/or sentences pronounced by the user which may be indicative of a cognitive decline. A linguistic parameter may for example indicate that the user is making lexical and/or grammatical simplifications, and/or loss of semantic abilities. The linguistic parameter may be determined based on a text transcript of the microphone input data. The text transcript may be obtained by automatic speech recognition (speech to text) algorithm or service. The linguistic parameter may comprise an embedding feature by a deep neural network (e.g. a BERT transformer network or other sequence-to-sequence autoencoders).

An acoustic parameter may be seen as a parameter indicative of an acoustic biomarker. In other words, an acoustic parameter may comprise specific acoustic sounds and/or acoustic features, such as acoustic sounds pronounced by the user and/or acoustic features in the voice of the user which may be indicative of a cognitive decline. An acoustic parameter may comprise one or more of: a pitch of the voice, an energy level, spectral parameters (mel-frequency cepstrum, MFCC; e.g. logMelSpec), spectral statistics (slope, roll-off-points), speech spectral envelope characteristics (e.g. formants, harmonics, ratios of harmonics and formants), and/or voice quality measures like harmonic to noise ratio, HNR, Jitter, and/or Shimmer.

A verbal fluency parameter may be seen as a parameter indicative of a verbal fluency biomarker. In other words, a verbal fluency parameter may comprise information about the verbal fluency of the user when the user is speaking. For example, when the verbal fluency of the user is decreasing and/or is indicative of a low verbal fluency, the verbal fluency parameter may be indicative of cognitive decline. The user of the hearing device may for example increasingly looking for words when speaking, which may be indicative of a decreasing verbal fluency. A verbal fluency parameter may for example indicate that the user is stuttering when speaking and/or has an increased stuttering when speaking. A verbal fluency parameter may for example indicate that the user is having circumlocutions and/or frequent use of filler sounds such as "uh, "um", semantic errors, indefinite terms, revisions, repetitions, and/or neologisms.

To determine the first voice biomarker may comprise to determine a coherence parameter. A coherence parameter may comprise information about the coherence of the user when the user is speaking. For example, when the coherence of the user speaking is decreasing and/or is indicative of a low coherence, the coherence parameter may be indicative of cognitive decline. The user of the hearing device may for example increasingly state implausible and/or irrelevant details.

It may be appreciated that using voice biomarkers is a simple and non-invasive was of characterizing and/or monitoring a health parameter.

The first criterion may comprise a first verbal fluency criterion. In other words, to determine whether the health parameter satisfies the first criterion may comprise to determine whether the health parameter satisfies the first verbal fluency criterion. For example, the first threshold may comprise a first verbal fluency threshold. When the verbal fluency parameter is below the first verbal fluency threshold, the first criterion is satisfied and the user may have a cognitive decline.

A mumbling parameter may be seen as a parameter indicative of a mumbling biomarker. In other words, a mumbling parameter may comprise information about the user mumbling when the user is speaking. For example, when the mumbling of the user may be increasing and/or be indicative of a high level of mumbling, the mumbling parameter may be indicative of cognitive decline. To determine a mumbling parameter may comprise to determine that a user is speaking quietly and indistinctly. In other words, to determine a mumbling parameter may comprise to determine that a user does not form his/her words clearly and/or that a user has an indistinct enunciation, e.g., with unintelligible speech and/or words.

The first criterion may comprise a first mumbling criterion. In other words, to determine whether the health parameter satisfies the first criterion may comprise to determine whether the health parameter satisfies the first mumbling criterion. For example, the first threshold may comprise a first mumbling threshold. When the mumbling parameter is above the first mumbling threshold, the first criterion is satisfied and the user may have a cognitive decline.

A voice pitch parameter may be seen as a parameter indicative of a voice pitch biomarker. In other words, a voice pitch parameter may comprise information about the voice pitch of the user when the user is speaking (such as pitch variation). For example, when the voice pitch of the user is increasing and/or decreasing, the voice pitch parameter may be indicative of cognitive decline.

The first criterion may comprise a first voice pitch criterion. In other words, to determine whether the health parameter satisfies the first criterion may comprise to determine whether the health parameter satisfies the first voice pitch criterion. For example, the first threshold may comprise a first voice pitch threshold. When the voice pitch parameter is above the first voice pitch threshold, the first criterion is satisfied and the user may have a cognitive decline.

A speech rhythm parameter may be seen as a parameter indicative of a speech rhythm biomarker. In other words, a speech rhythm parameter may comprise information about the speech rhythm of the user when the user is speaking. For example, when the speech rhythm of the user is decreasing, the speech rhythm parameter may be indicative of cognitive decline. Another example may be that the speech rhythm is indicative of the user hesitating when speaking.

The first criterion may comprise a first speech rhythm criterion. In other words, to determine whether the health parameter satisfies the first criterion may comprise to determine whether the health parameter satisfies the first speech rhythm criterion. For example, the first threshold may comprise a first speech rhythm threshold. When the speech rhythm parameter is above the first speech rhythm threshold, the first criterion is satisfied and the user may have a cognitive decline.

The pitch feature and the speech rhythm feature may be seen as prosodic features.

In one or more example hearing systems, electronic devices, and/or hearing devices, the sensor data, such as external sensor data, comprises physiological data. In one or more example hearing systems, electronic devices, and/or hearing devices, the health parameter is based on the physiological data.

In one or more example hearing systems and/or electronic devices, the one or more sensors comprise one or more physiological sensors for provision of physiological data. A physiological sensor may for example comprise one or more of: a body sensor, e.g., a heart rate sensor, a blood pressure sensor, a pulse sensor, a photoplethysmogram (PPG) sensor, an electrocardiogram (ECG) sensor, an electroencephalogram (EEG) sensor, a bioimpedance sensor, and a temperature sensor. The one or more sensors of the hearing device may comprise a body sensor being a capacitive and/or conductivity sensor, e.g. to measure and/or determine stress levels.

In one or more example hearing systems, electronic devices, and/or hearing devices, to determine a health parameter comprises to determine, based on the physiological data, a first physiological biomarker. To determine a health parameter may for example comprise to determine a second physiological biomarker, a third physiological biomarker, a fourth physiological biomarker, a fifth physiological biomarker, and/or a sixth physiological biomarker. To determine a physiological biomarker, such as the first physiological biomarker, may comprise to extract one or more features, such as physiological features, from the sensor data. To determine a physiological biomarker, such as the first physiological biomarker, may comprise to extract one or more physiological features from the physiological data. A physiological biomarker may be seen as a biomarker indicative of one or more parameters of a physiological condition the user of the hearing device. A physiological biomarker may comprise and/or be indicative of a signature, such as a physiological signature, in the physiological data. The physiological data may be indicative of and/or be representative of one or more physiological processes. For example, the physiological data may be indicative of and/or be representative of one or more physiological processes, such as changes in the physiological processes of the user. A change in the physiological processes of the user may for example comprise changes in the throat and/or chest as well as changes to the nerves which are supposed to control a physiological process. The physiological processes may be affected by cognitive decline and the changes in the physiological processes may be identified and/or detected as cognitive decline of the user.

In one or more example hearing systems, electronic devices, and/or hearing devices, the first criterion is based on the first physiological biomarker. In other words, the first criterion comprises a first physiological biomarker criterion. In other words, to determine whether the health parameter satisfies the first criterion may comprise to determine whether the health parameter satisfies the first physiological biomarker criterion.

In one or more example hearing systems, electronic devices, and/or hearing devices, to determine a first physiological biomarker comprises to determine one or more of: a blood pressure parameter, a blood flow parameter, a heart rate parameter, a respiratory parameter, a temperature parameter, an oxygen parameter, and a brain activity parameter. In one or more example hearing systems, the system is configured, such as using the electronic device and/or the hearing device, to determine one or more of: a blood pressure parameter, a blood flow parameter, a heart rate parameter, a respiratory parameter, a temperature parameter, an oxygen parameter, and a brain activity parameter.

In one or more example electronic devices, the processor of the electronic device is configured to determine one or more of: a blood pressure parameter, a blood flow parameter, a heart rate parameter, a respiratory parameter, a temperature parameter, an oxygen parameter, and a brain activity parameter.

In one or more example hearing devices, the processor of the hearing device is configured to determine one or more of: a blood pressure parameter, a blood flow parameter, a heart rate parameter, a respiratory parameter, a temperature parameter, an oxygen parameter, and a brain activity parameter.

In one or more example hearing systems, electronic devices, and/or hearing devices, the first physiological biomarker is based on one or more of: the blood pressure parameter, the blood flow parameter, the heart rate parameter, the respiratory parameter, the temperature parameter, the oxygen parameter, and the brain activity parameter.

A blood pressure parameter may be seen as a parameter indicative of a blood pressure biomarker. In other words, a blood pressure parameter may comprise information about the blood pressure of the user when the user is wearing the hearing device. For example, when the blood pressure of the user is changing abnormally, such as increasing and/or decreasing, the blood pressure parameter may be indicative of cognitive decline and/or indicative of an increased risk of cognitive decline.

The first criterion may comprise a first blood pressure criterion. In other words, to determine whether the health parameter satisfies the first criterion may comprise to determine whether the health parameter satisfies the first blood pressure criterion. For example, the first threshold may comprise a first blood pressure threshold and/or a second blood pressure threshold. When the blood pressure parameter is above the first blood pressure threshold and/or below the second blood pressure threshold, the first criterion is satisfied, and the user may have a cognitive decline and/or indicative of an increased risk of cognitive decline.

A blood flow parameter may be seen as a parameter indicative of a blood flow biomarker. In other words, a blood flow parameter may comprise information about the blood flow of the user when the user is wearing the hearing device. For example, when the blood flow of the user is changing abnormally, such as increasing and/or decreasing, the blood flow parameter may be indicative of cognitive decline and/or indicative of an increased risk of cognitive decline.

The first criterion may comprise a first blood flow criterion. In other words, to determine whether the health parameter satisfies the first criterion may comprise to determine whether the health parameter satisfies the first blood flow criterion. For example, the first threshold may comprise a first blood flow threshold and/or a second blood flow threshold. When the blood flow parameter is above the first blood flow threshold and/or below the second blood flow threshold, the first criterion is satisfied, and the user may have a cognitive decline and/or the user may have an increased risk of cognitive decline.

A heart rate parameter may be seen as a parameter indicative of a heart rate biomarker. In other words, a heart rate parameter may comprise information about the heart rate of the user when the user is wearing the hearing device. For example, when the heart rate of the user is changing abnormally, such as increasing and/or decreasing, the heart rate parameter may be indicative of cognitive decline. A heart rate parameter may be indicative of a heart rate variability.

The first criterion may comprise a first heart rate criterion. In other words, to determine whether the health parameter satisfies the first criterion may comprise to determine whether the health parameter satisfies the first heart rate criterion. For example, the first threshold may comprise a first heart rate threshold and/or a second heart rate threshold. When the heart rate parameter is above the first heart rate threshold and/or below the second heart rate threshold, the first criterion is satisfied, and the user may have a cognitive decline and/or the user may have an increased risk of cognitive decline.

A respiratory parameter may be seen as a parameter indicative of a respiratory biomarker. In other words, a respiratory parameter may comprise information about the respiratory conditions of the user when the user is wearing the hearing device. For example, when a respiratory rate of the user is changing abnormally, such as increasing and/or decreasing, the respiratory parameter may be indicative of cognitive decline and/or indicative of an increased risk of cognitive decline.

The first criterion may comprise a first respiratory criterion. In other words, to determine whether the health parameter satisfies the first criterion may comprise to determine whether the health parameter satisfies the first respiratory criterion. For example, the first threshold may comprise a first respiratory rate threshold and/or a second respiratory rate threshold. When the respiratory parameter is above the first respiratory rate threshold and/or below the second respiratory rate threshold, the first criterion is satisfied, and the user may have a cognitive decline and/or the user may have an increased risk of cognitive decline.

A temperature parameter may be seen as a parameter indicative of a temperature biomarker. In other words, a temperature parameter may comprise information about the temperature of the user when the user is wearing the hearing device. For example, when the temperature of the user is changing abnormally, such as increasing and/or decreasing, the temperature parameter may be indicative of cognitive decline and/or the user may have an increased risk of cognitive decline.

The first criterion may comprise a first temperature criterion. In other words, to determine whether the health parameter satisfies the first criterion may comprise to determine whether the health parameter satisfies the first temperature criterion. For example, the first threshold may comprise a first temperature threshold and/or a second temperature threshold. When the temperature parameter is above the first temperature threshold and/or below the second temperature threshold, the first criterion is satisfied, and the user may have a cognitive decline and/or the user may have an increased risk of cognitive decline.

An oxygen parameter may be seen as a parameter indicative of an oxygen biomarker. In other words, an oxygen parameter may comprise information about the oxygen of the user when the user is wearing the hearing device. For example, when an oxygen rate of the user is changing abnormally, such as increasing and/or decreasing, the oxygen parameter may be indicative of cognitive decline and/or the user may have an increased risk of cognitive decline.

The first criterion may comprise a first oxygen criterion. In other words, to determine whether the health parameter satisfies the first criterion may comprise to determine whether the health parameter satisfies the first oxygen criterion. For example, the first threshold may comprise a first oxygen threshold. When the oxygen parameter is below the first oxygen threshold, the first criterion is satisfied, and the user may have a cognitive decline and/or the user may have an increased risk of cognitive decline.

A brain activity parameter may be seen as a parameter indicative of a brain activity biomarker. In other words, a brain activity parameter may comprise information about the brain activity of the user when the user is wearing the hearing device. For example, when the brain activity of the user is changing abnormally, such as increasing and/or decreasing, the brain activity parameter may be indicative of cognitive decline and/or indicative of an increased risk of cognitive decline.

The first criterion may comprise a first brain activity criterion. In other words, to determine whether the health parameter satisfies the first criterion may comprise to determine whether the health parameter satisfies the first brain activity criterion. For example, the first threshold may comprise a first brain activity threshold. When the brain activity parameter is below the first brain activity threshold, the first criterion is satisfied, and the user may have a cognitive decline and/or the user may have an increased risk of cognitive decline.

In one or more example hearing systems, electronic devices, and/or hearing devices, the sensor data, such as external sensor data, comprises biokinetic data. In one or more example hearing systems, electronic devices, and/or hearing devices, the health parameter is based on the biokinetic data.

In one or more example hearing systems and/or electronic devices, the one or more sensors comprise one or more biokinetic sensors for provision of biokinetic data. A biokinetic sensor may for example comprise one or more of: a motion sensor, an IMU sensor, an accelerometer, and/or a gyroscope. The biokinetic sensor(s) may be configured to measure one or more movements of the user of the hearing device. For example, the biokinetic sensor(s) may be configured to measure one or more head movements of the user of the hearing device.

In one or more example hearing systems, electronic devices, and/or hearing devices, to determine a health parameter comprises to determine, based on the biokinetic data, a first biokinetic biomarker. To determine a health parameter may for example comprise to determine a second biokinetic biomarker, a third biokinetic biomarker, a fourth biokinetic biomarker, a fifth biokinetic biomarker, and/or a sixth biokinetic biomarker. To determine a biokinetic biomarker, such as the first biokinetic biomarker, may comprise to extract one or more features, such as biokinetic features, from the sensor data. To determine a biokinetic biomarker, such as the first biokinetic biomarker, may comprise to extract one or more biokinetic features from the biokinetic data. A biokinetic biomarker may be seen as a biomarker indicative of one or more movements of the user of the hearing device. A biokinetic biomarker may comprise and/or be indicative of a signature, such as a biokinetic signature, in the biokinetic data.

In one or more example hearing systems, electronic devices, and/or hearing devices, the first criterion is based on the first biokinetic biomarker. In other words, the first criterion comprises a first biokinetic biomarker criterion. In other words, to determine whether the health parameter satisfies the first criterion may comprise to determine whether the health parameter satisfies the first biokinetic biomarker criterion.

In one or more example hearing systems, electronic devices, and/or hearing devices, to determine a first biokinetic biomarker comprises to determine one or more of: a motion parameter, a trembling parameter, a shaking parameter, and a tic parameter.

In one or more example hearing systems, the system is configured, such as using the electronic device and/or the hearing device, to determine one or more of: a motion parameter, a trembling parameter, a shaking parameter, and a tic parameter.

In one or more example electronic devices, the processor of the electronic device is configured to determine one or more of: a motion parameter, a trembling parameter, a shaking parameter, and a tic parameter.

In one or more example hearing devices, the processor of the hearing device is configured to determine one or more of: a motion parameter, a trembling parameter, a shaking parameter, and a tic parameter.

In one or more example hearing systems, electronic devices, and/or hearing devices, the first biokinetic biomarker is based on one or more of: the motion parameter, the trembling parameter, the shaking parameter, and the tic parameter.

A motion parameter may be seen as a parameter indicative of a motion biomarker. In other words, a motion parameter may comprise information about the motion of the user when the user is wearing the hearing device. For example, when the motion of the user is changing abnormally, such as increasing and/or decreasing, the motion parameter may be indicative of cognitive decline.

A trembling parameter may be seen as a parameter indicative of a trembling biomarker. In other words, a trembling parameter may comprise information about the trembling of the user when the user is wearing the hearing device. For example, when the trembling of the user is changing abnormally, such as increasing and/or decreasing, the trembling parameter may be indicative of cognitive decline.

A shaking parameter may be seen as a parameter indicative of a shaking biomarker. In other words, a shaking parameter may comprise information about the shaking of the user when the user is wearing the hearing device. For example, when the shaking of the user is changing abnormally, such as increasing, the shaking parameter may be indicative of cognitive decline.

A tic parameter may be seen as a parameter indicative of a tic biomarker. In other words, a tic parameter may comprise information about one or more tics of the user when the user is wearing the hearing device. For example, when the tic(s) of the user is changing abnormally, such as increasing, the tic parameter may be indicative of cognitive decline.

It is to be understood that a description of a feature in relation to the hearing device and/or the electronic device(s) is also applicable to the corresponding feature in the system(s), and/or the method(s) of operating a hearing system, the method(s) for operating a hearing device, and/or the method(s) for operating an electronic device as disclosed herein.

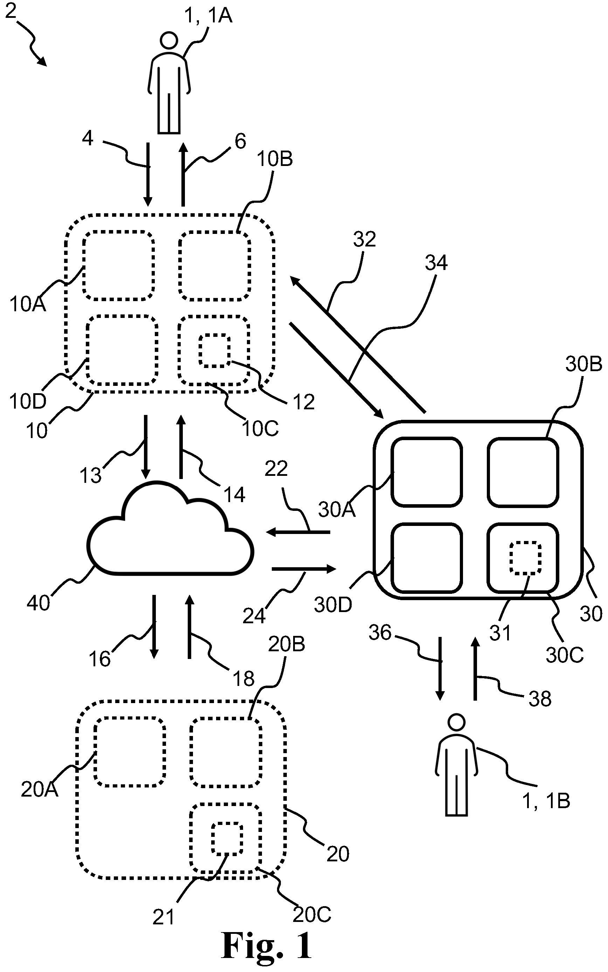

Fig. 1 schematically illustrates an example hearing system, such as a hearing system 2 according to the present disclosure. The hearing system 2 comprises an electronic device 10 comprising a memory 10A, an interface 10B, and a processor 10C. The hearing system 2 comprises a hearing device 30 comprising a memory 30A, an interface 30A, a processor 30C, and one or more sensors 30D.

The electronic device 10 comprises a memory 10A, an interface 10B (such as one or more interfaces), and a processor 10C.

Optionally, the electronic device is a server device 20. In other words, the hearing system 2 may comprise a server device 20. The server device 20 comprises a memory 20A, an interface 20B (such as one or more interfaces), and a processor 20C (such as one or more processors).

Optionally, the system 2 comprises machine learning circuitry configured to operate according to a machine learning model. In one or more example hearing systems, to determine a health parameter comprises to determine, such as using the electronic device 10, 20, and/or the hearing device 30, the health parameter, based on the external sensor data, using the machine learning model. In other words, the electronic device 10, 20, and/or the hearing device 30 may comprise machine learning circuitry configured to operate according to a machine learning model. In other words, the system 2 may be configured to determine, such as using the electronic device 10, 20 and/or the hearing device 30, the health parameter, based on the external sensor data, using the machine learning model.

Optionally, the processor 10C may comprise machine learning circuitry 12 configured to operate according to a machine learning model. Optionally, the processor 20C may comprise machine learning circuitry 21 configured to operate according to a machine learning model. Optionally, the processor 30C may comprise machine learning circuitry 31 configured to operate according to a machine learning model. In one or more example electronic devices and/or systems, the model as referred to herein may be stored on a non-transitory storage medium (for example, on the memory 10A of the electronic device 10, on the memory 20A of the server device 20, and/or on the memory 30A of the hearing device 30).

Optionally, the one or more sensors 10D and/or one or more sensors 30D comprise a microphone for provision of microphone input data. Optionally, the external sensor data comprises microphone input data.

Optionally, the health parameter is based on the microphone input data.

Optionally, the one or more sensors 30D and/or the one or more sensors 30D comprise one or more of: a microphone, an optical sensor, a magnetic sensor, a galvanic sensor, a motion sensor, and a capacitive sensor.

Optionally, the interface 10B of the electronic device 10 and/or the interface 30B of the hearing device 30 comprises one or more of: a Bluetooth interface, Bluetooth low energy interface, and a magnetic induction interface. For example, the interface 10B of the electronic device 10 and/or the interface 30B of the hearing device 30 may comprise a Bluetooth antenna and/or a magnetic interference antenna.

The system 2 is configured to obtain external sensor data from the one or more sensors, such as one or more sensors 10D and/or one or more sensors 30D. In other words, the system 2 is configured to obtain the external sensor data at the hearing device 30 and/or at the electronic device acting as accessory device 10 and/or electronic device acting as server device 20. In other words, the external sensor data may be obtained, received, and/or retrieved by the electronic device acting as accessory device 10 and/or electronic device acting as server device 20 from the hearing device 30. The external sensor data may be transmitted and/or sent by the hearing device 30 to the electronic device acting as accessory device 10 and/or electronic device acting as server device 20. The system 2 is configured to determine, such as using the processor 30C of the hearing device 30 and/or the processor 10C of the electronic device acting as accessory device 10 and/or electronic device acting as server device 20, based on the external sensor data, a health parameter indicative of a cognitive state of a user 1, 1B of the hearing device 30. In other words, the system 2 is configured to determine, such as at the processor 30C of the hearing device 30 and/or at the processor 10C of the electronic device acting as accessory device 10 and/or the processor 20C of the electronic device acting as server device 20, based on the external sensor data, a health parameter indicative of a cognitive state of a user 1, 1B of the hearing device 30. The system 2 is configured to determine, such as using the processor 30C of the hearing device 30 and/or the processor 10C of the electronic device acting as accessory device 10 and/or electronic device acting as server device 20, whether the health parameter satisfies a first criterion indicative of a cognitive decline. In other words, the system 2 is configured to determine, such as at the processor 30C of the hearing device 30 and/or the processor 10C of the electronic device acting as accessory device 10 and/or electronic device acting as server device 20, whether the health parameter satisfies a first criterion indicative of a cognitive decline. Optionally, the system 2 is configured, in accordance with the health parameter satisfying the first criterion, to output, such as using the processor 30C of the hearing device 30 and/or the processor 10C of the electronic device acting as accessory device 10 and/or electronic device acting as server device 20, via the interface 30B of the hearing device 30 and/or the interface 10B of the electronic device acting as accessory device 10 and/or the interface 20B of the electronic device acting as server device 20, a health representation associated with the cognitive state.

A hearing device 30 is disclosed. The hearing device 30 may be seen as a hearing device with health characterization and/or monitoring. The hearing device 30 comprises a memory 30A, an interface 30B, a processor 30C, and one or more sensors 30D. The processor 30C is configured to obtain sensor data from the one or more sensors 30D. Optionally, the processor 30C is configured to obtain 24, 34 internal sensor data from the one or more sensors 10D of the electronic device 10, e.g, via the network 40 such as a global network, e.g. the internet, and/or a local network. The processor 30C is configured to determine, based on the sensor data, a health parameter indicative of a cognitive state of a user 1, 1B of the hearing device 30. The processor 30C is configured to determine whether the health parameter satisfies a first criterion indicative of a cognitive decline. Optionally, the processor 30C is configured, in accordance with the health parameter satisfying the first criterion, to output 22, 36, via the interface 30B, a health representation associated with the cognitive state. Optionally, the user 1, 1B may provide an input 38 (such as user input), such as via the interface 30B, to the hearing device 30. The determination of one or more of the sensor data, the health parameter, and the health representation may be based on the input 38 from the user 1, 1B. The user 1, 1B may for example provide one or more inputs in response to a cognitive test scheme.

An electronic device acting as an accessory device 10 is disclosed. The electronic device 10 comprises a memory 10A, an interface 10B, and a processor 10C. The processor 10C is configured to obtain 14, 32, via the interface 10B, (such as via the network 40 such as a global network, e.g. the internet, and/or a local network) external sensor data from a hearing device, such as hearing device 30. The processor 10C is configured to determine, based on the external sensor data, a health parameter indicative of a cognitive state of a user 1, 1B of the hearing device 30. The processor 10C is configured to determine whether the health parameter satisfies a first criterion indicative of a cognitive decline. Optionally, the processor 10C is configured, in accordance with the health parameter satisfying the first criterion, to output 6, 34 via the interface 10B, a health representation associated with the cognitive state.

An electronic device acting as an server device 20 is disclosed. The electronic device 20 comprises a memory 20A, an interface 20B, and a processor 20C. The processor 20C is configured to obtain 16, via the interface 20B, (such as via the network 40 such as a global network, e.g. the internet, and/or a local network) external sensor data from a hearing device, such as hearing device 30. The processor 20C is configured to determine, based on the external sensor data, a health parameter indicative of a cognitive state of a user 1, 1B of the hearing device 30. The processor 20C is configured to determine whether the health parameter satisfies a first criterion indicative of a cognitive decline. Optionally, the processor 20C is configured, in accordance with the health parameter satisfying the first criterion, to output 18, via the interface 20B, (such as via network 40, to the electronic device 10) a health representation associated with the cognitive state.

Optionally, the electronic device 10 comprises one or more sensors 10D, such as one or more internal sensors. In one or more hearing systems, hearing devices, and/or electronic devices, the system 2 is configured to obtain internal sensor data from the one or more sensors 10D of the electronic device 10. In one or more hearing systems, hearing devices, and/or electronic devices, the health parameter is based on the internal sensor data. The internal sensor data may be seen as sensor data obtained from the electronic device 10. In other words, the internal sensor data may be seen as sensor data generated by the one or more sensors 10D of the electronic device 10. It may be appreciated that the health parameter is determined based on the sensor data (such as external sensor data) and/or the internal sensor data.

Optionally, the system 2, such as the server device 20, is configured to train the machine learning model, to provide an updated machine learning model, and to transmit 14, 18, 24 to the electronic device 10 and/or the hearing device 30, the updated machine learning model. In other words, the server device 20 is configured to train the machine learning model, to provide an updated machine learning model, and to transmit 18, to the electronic device 10 and/or the hearing device 30, the updated machine learning model.

Optionally, to output the health representation comprises to display 6, via the interface 10B of the electronic device 10, a user interface representing the health representation. In other words, to output the health representation comprises to display 6, via the interface 10B of the accessory device 10 as disclosed herein, a user interface representing the health representation, e.g., to a user 1, 1A.

Optionally, in accordance with the first criterion being satisfied, the system 2 is configured to perform, via the interface 10B of the electronic device 10, a cognitive test scheme. In other words, in accordance with the first criterion being satisfied, the system 2 is configured to perform, via the interface 10B of the accessory device 10, a cognitive test scheme. In one or more example hearing systems and/or electronic devices, in accordance with the first criterion being satisfied, the system 2 is configured to perform, via the interface 30B of the hearing device 30, a cognitive test scheme.

Optionally, the cognitive test scheme may be comprised in a health representation and/or the health representation may be based on the cognitive test scheme.

Optionally, the system 2 is configured, such as using the electronic device 10, 20 and/or the hearing device 30, to determine a health parameter comprises to determine, based on the microphone input data, a first voice biomarker.

Optionally, the system 2 is configured, such as using the electronic device 10, 20 and/or the hearing device 30, to determine one or more of: a linguistic parameter, an acoustic parameter, a verbal fluency parameter, a mumbling parameter, a voice pitch parameter, and a speech rhythm parameter.

Optionally, the one or more sensors 10D, 30D comprise one or more physiological sensors for provision of physiological data.

Optionally, the sensor data, such as external sensor data, comprises physiological data, such as physiological data associated with the user 1, 1B. In one or more example hearing systems, electronic devices, and/or hearing devices, the health parameter is based on the physiological data.

Optionally, the system 2 is configured, such as using the electronic device 10, 20 and/or the hearing device 30, to determine, based on the physiological data, a first physiological biomarker.

Optionally, the system 2 is configured, such as using the electronic device 10, 20 and/or the hearing device 30, to determine one or more of: a blood pressure parameter, a blood flow parameter, a heart rate parameter, a respiratory parameter, a temperature parameter, an oxygen parameter, and a brain activity parameter.

Optionally, the system 2 is configured, such as using the electronic device 10, 20 and/or the hearing device 30, to determine one or more of: a blood pressure parameter, a blood flow parameter, a heart rate parameter, a respiratory parameter, a temperature parameter, an oxygen parameter, and a brain activity parameter.

Optionally, the one or more sensors 10D, 30D comprise one or more biokinetic sensors for provision of biokinetic data.

Optionally, the sensor data, such as external sensor data, comprises biokinetic data, such as biokinetic data associated with the user 1, 1B.

Optionally, the system 2 is configured, such as using the electronic device 10, 20 and/or the hearing device 30, to determine, based on the biokinetic data, a first biokinetic biomarker.

Optionally, the system 2 is configured, such as using the electronic device 10, 20 and/or the hearing device 30, to determine one or more of: a motion parameter, a trembling parameter, a shaking parameter, and a tic parameter.

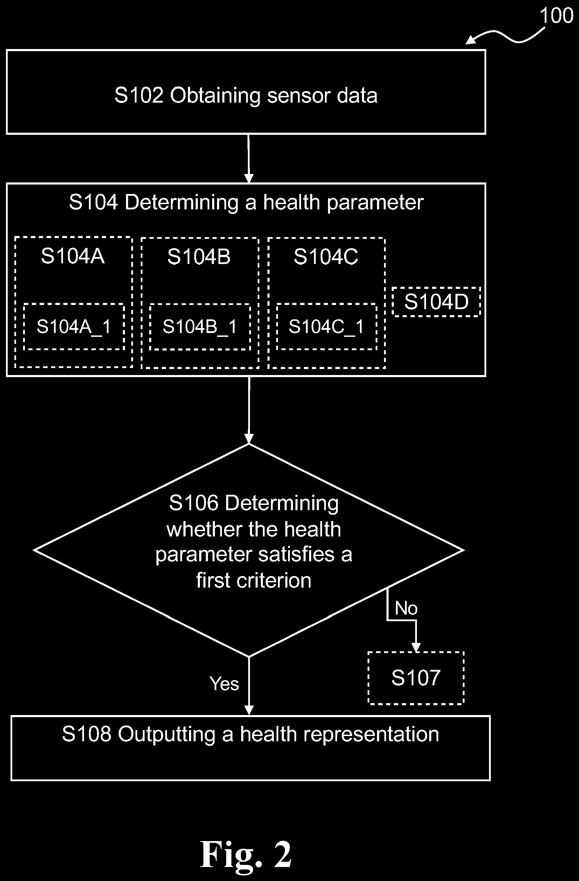

The hearing device 30 may be configured to perform any of the methods disclosed in Fig. 2.

The processor 30C is optionally configured to perform any of the operations disclosed in Fig. 2 (such as any one or more of S104A, S104A_1, S104B, S104B_1, S104C, S104C_1, S104D, S107). The operations of the hearing device 30 may be embodied in the form of executable logic routines (for example, lines of code, software programs, etc.) that are stored on a non-transitory computer readable medium (for example, memory 30A) and are executed by the processor 30C).

Furthermore, the operations of the hearing device 30 may be considered a method that the hearing device 10 is configured to carry out. Also, while the described functions and operations may be implemented in software, such functionality may as well be carried out via dedicated hardware or firmware, or some combination of hardware, firmware and/or software.

Memory 30A may be one or more of a buffer, a flash memory, a hard drive, a removable media, a volatile memory, a non-volatile memory, a random access memory (RAM), or other suitable device. In a typical arrangement, memory 30A may include a non-volatile memory for long term data storage and a volatile memory that functions as system memory for the processor 30C. The memory 10A may exchange data with the processor 30C over a data bus. Control lines and an address bus between the memory 30B and the processor 30C also may be present (not shown in Fig. 1). The memory 30A is considered a non-transitory computer readable medium.

The memory 30A may be configured to store information such as sensor data, sound data, audio data, image data, health parameter(s), health representation(s), biomarker(s), recommendation(s), and/or machine learning model(s) as disclosed herein in a part of the memory.

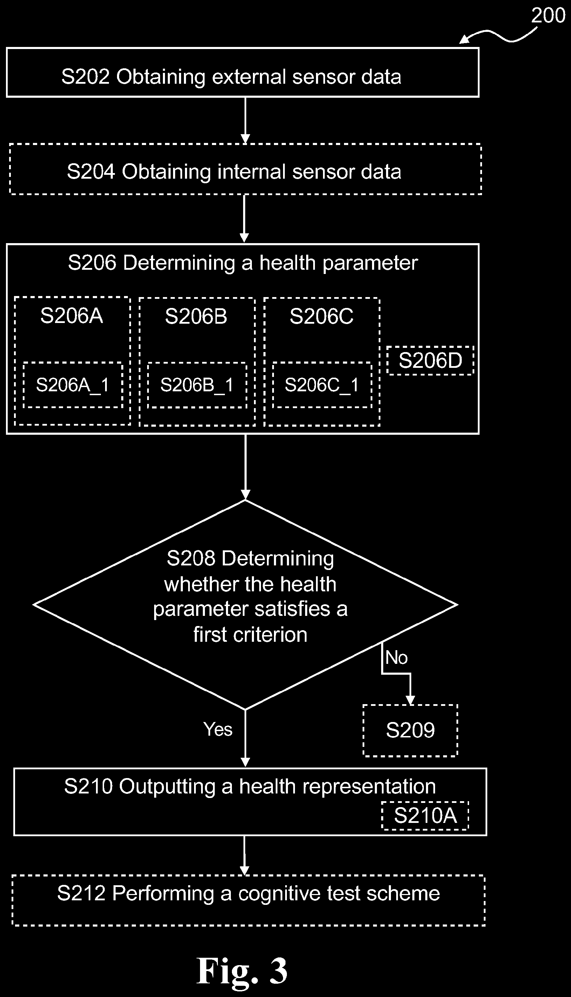

The electronic device 10 may be configured to perform any of the methods disclosed in Fig. 3.

The processor 10C is optionally configured to perform any of the operations disclosed in Fig. 2A (such as any one or more of S204, S206A, S206A_1, S206B, S206B_1, S206C, S206C_1, S206D, S209, S210A, S212). The operations of the electronic device 10 may be embodied in the form of executable logic routines (for example, lines of code, software programs, etc.) that are stored on a non-transitory computer readable medium (for example, memory 10A) and are executed by the processor 10C).

Furthermore, the operations of the electronic device 10 may be considered a method that the electronic device 10 is configured to carry out. Also, while the described functions and operations may be implemented in software, such functionality may as well be carried out via dedicated hardware or firmware, or some combination of hardware, firmware and/or software.

Memory 10A may be one or more of a buffer, a flash memory, a hard drive, a removable media, a volatile memory, a non-volatile memory, a random access memory (RAM), or other suitable device. In a typical arrangement, memory 10A may include a non-volatile memory for long term data storage and a volatile memory that functions as system memory for the processor 10C. The memory 10A may exchange data with the processor 10C over a data bus. Control lines and an address bus between the memory 10B and the processor 10C also may be present (not shown in Fig. 1). The memory 10A is considered a non-transitory computer readable medium.

The memory 10A may be configured to store information such as sensor data, sound data, audio data, image data, health parameter(s), health representation(s), biomarker(s), recommendation(s), and/or machine learning model(s) as disclosed herein in a part of the memory.

The server device 20, such as the processor 20C, may be configured to perform any of the operations performed by the electronic device 10, such as accessory device 10, such as the processor 10C, as described herein. In other words, the description related to the processor 10C may apply to the description of the processor 20C. For example, when the electronic device acts as a server device, the electronic device 10 and the server device 20 may be considered as one device and/or two separate devices.

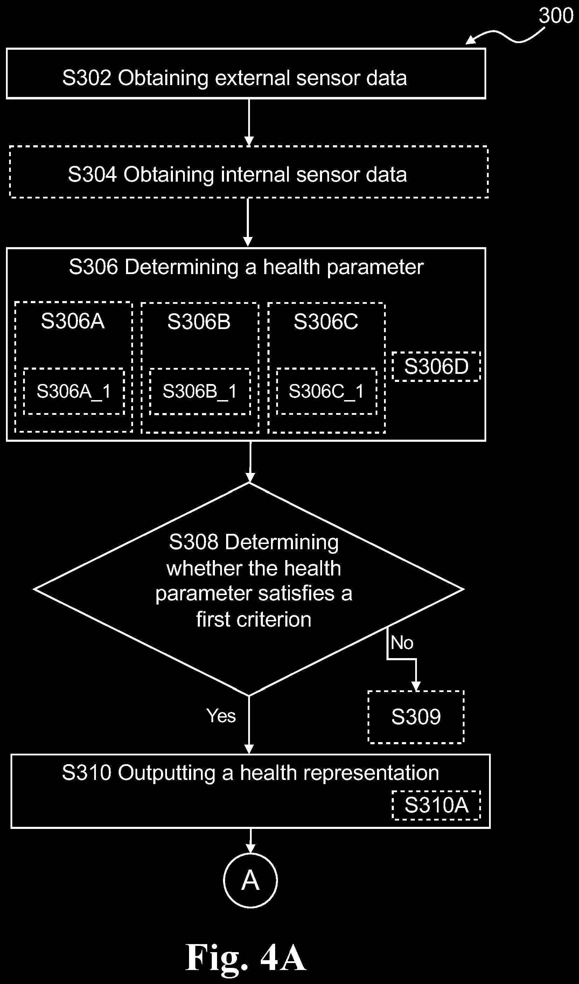

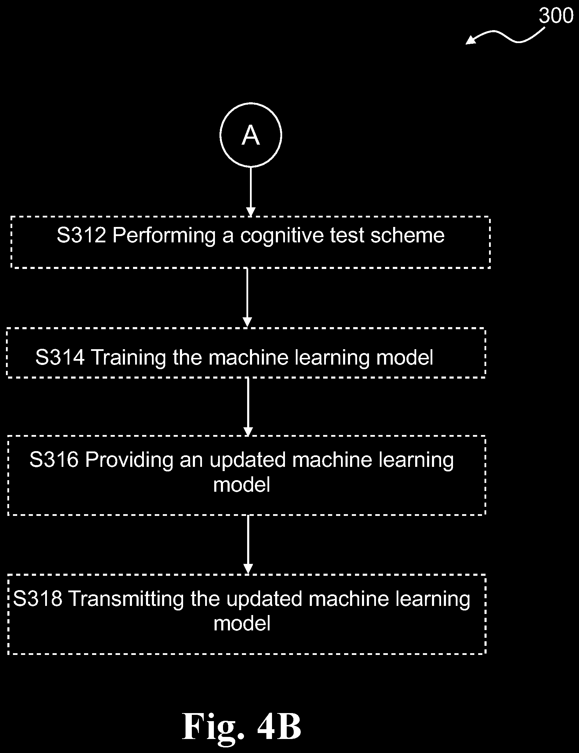

The system 2 may be configured to perform any of the methods disclosed in Figs. 4A-4B.

The system 2 is optionally configured to perform any of the operations disclosed in Figs. 4A-4B (such as any one or more of S304, S306A, S306A_1, S306B, S306B_1, S306C, S306C_1, S306D, S309, S310A, S312, S314, S316, S318). The operations of the system 2 may be embodied in the form of executable logic routines (for example, lines of code, software programs, etc.) that are stored on a non-transitory computer readable medium (for example, memory 10A, 30A) and are executed by the processor 10C, 30C).

Furthermore, the operations of the system 2 may be considered a method that the system 2 is configured to carry out. Also, while the described functions and operations may be implemented in software, such functionality may as well be carried out via dedicated hardware or firmware, or some combination of hardware, firmware and/or software.

Fig. 2 shows a flow diagram of an example method, such as a method 100. A method 100 of operating a hearing device is disclosed. The method 100, may be performed by a hearing device as disclosed herein, such as hearing device 30.

The method 100 comprises obtaining S102, from one or more sensors of the hearing device, sensor data.

The method 100 comprises determining S104, based on the sensor data, a health parameter indicative of a cognitive state of a user of the hearing device. The method 100 comprises determining S106 whether the health parameter satisfies a first criterion indicative of a cognitive decline. The method 100 comprises, in accordance with the health parameter satisfying the first criterion, outputting S108 a health representation associated with the cognitive state.

Optionally, in accordance with the health parameter not satisfying the first criterion, the method 100 comprises refraining S107 from outputting S108 a health representation associated with the cognitive state.

In one or more example methods, the one or more sensors comprise a microphone for provision of microphone input data. In one or more example methods, the health parameter is based on the microphone input data.

In one or more example methods, determining S104 a health parameter comprises determining S104A, based on the microphone input data, a first voice biomarker. In one or more example methods, the first criterion is based on the first voice biomarker.

In one or more example methods, determining S104A the first voice biomarker comprises determining S104A_1 one or more of: a linguistic parameter, an acoustic parameter, a verbal fluency parameter, a mumbling parameter, a voice pitch parameter, and a speech rhythm parameter. In one or more example methods, the first voice biomarker is based on one or more of: a linguistic parameter, an acoustic parameter, a verbal fluency parameter, a mumbling parameter, a voice pitch parameter, and a speech rhythm parameter.

In one or more example methods, the one or more sensors comprise one or more physiological sensors for provision of physiological data. In one or more example methods, the health parameter is based on the physiological data.

In one or more example methods, determining S104 a health parameter comprises determining S104B, based on the physiological data, a first physiological biomarker. In one or more example methods, the first criterion is based on the first physiological biomarker.

In one or more example methods, determining S104B a first physiological biomarker comprises determining S104B_1 one or more of: a blood pressure parameter, a blood flow parameter, a heart rate parameter, a respiratory parameter, a temperature parameter, an oxygen parameter, and a brain activity parameter. In one or more example methods, the first physiological biomarker is based on one or more of: the blood pressure parameter, the blood flow parameter, the heart rate parameter, the respiratory parameter, the temperature parameter, the oxygen parameter, and the brain activity parameter.

In one or more example methods, the one or more sensors comprise one or more biokinetic sensors for provision of biokinetic data. In one or more example methods, the health parameter is based on the biokinetic data.

In one or more example methods, determining S104 a health parameter comprises determining S104C, based on the biokinetic data, a first biokinetic biomarker. In one or more example methods the first criterion is based on the first biokinetic biomarker.

In one or more example methods, determining S104C a first biokinetic biomarker comprises determining S104C_1 one or more of: a motion parameter, a trembling parameter, a shaking parameter, and a tic parameter. In one or more example methods, the first biokinetic biomarker is based on one or more of: a motion parameter, a trembling parameter, a shaking parameter, and a tic parameter.

In one or more example methods, the one or more sensors comprise one or more of: an optical sensor, a magnetic sensor, a galvanic sensor, a motion sensor, and a capacitive sensor.

In one or more example methods, the processor comprises machine learning circuitry configured to operate according to a machine learning model. In one or more example methods determining S104 a health parameter comprises determining S104D the health parameter, based on the sensor data, using the machine learning model.

Fig. 3 shows a flow diagram of an example method, such as a method 200. A method 200 of operating an electronic device is disclosed. The method 200, may be performed by an electronic device as disclosed herein, such as electronic device 10.

A method 200 of operating an electronic device is disclosed. The method 200 comprises obtaining S202 external sensor data from a hearing device. The method 200 comprises determining S206, based on the external sensor data, a health parameter indicative of a cognitive state of a user of the hearing device. The method 200 comprises determining S208 whether the health parameter satisfies a first criterion indicative of a cognitive decline. The method 200 comprises, in accordance with the health parameter satisfying the first criterion, outputting S210 a health representation associated with the cognitive state.

Optionally, in accordance with the health parameter not satisfying the first criterion, the method 200 comprises refraining S209 from outputting S210 a health representation associated with the cognitive state.

In one or more example methods, outputting S210 the health representation comprises displaying S210A a user interface representing the health representation.

In one or more example methods, in accordance with the first criterion being satisfied, the method comprises performing S212 a cognitive test scheme.

In one or more example methods, the external sensor data comprises microphone input data. In one or more example methods, the health parameter is based on the microphone input data.

In one or more example methods, determining S206 a health parameter comprises determining S206A, based on the microphone input data, a first voice biomarker. In one or more example methods, the first criterion is based on the first voice biomarker.

In one or more example methods, determining S206A the first voice biomarker comprises determining S206A_1 one or more of: a linguistic parameter, an acoustic parameter, a verbal fluency parameter, a mumbling parameter, a voice pitch parameter, and a speech rhythm parameter. In one or more example methods, the first voice biomarker is based on one or more of: a linguistic parameter, an acoustic parameter, a verbal fluency parameter, a mumbling parameter, a voice pitch parameter, and a speech rhythm parameter.

In one or more example methods, the external sensor data comprises physiological data. In one or more example methods, the health parameter is based on the physiological data.

In one or more example methods, determining S206 a health parameter comprises determining 206B, based on the physiological data, a first physiological biomarker. In one or more example methods, the first criterion is based on the first physiological biomarker.

In one or more example methods, determining S206B a first physiological biomarker comprises determining S206B_1 one or more of: a blood pressure parameter, a blood flow parameter, a heart rate parameter, a respiratory parameter, a temperature parameter, an oxygen parameter, and a brain activity parameter. In one or more example methods, the first physiological biomarker is based on one or more of: the blood pressure parameter, the blood flow parameter, the heart rate parameter, the respiratory parameter, the temperature parameter, the oxygen parameter, and the brain activity parameter.

In one or more example methods, the external sensor data comprises biokinetic data. In one or more example methods, the health parameter is based on the biokinetic data.

In one or more example methods, determining S206 a health parameter comprises determining S206C, based on the biokinetic data, a first biokinetic biomarker. In one or more example methods, the first criterion is based on the first biokinetic biomarker.

In one or more example methods, determining S206C a first biokinetic biomarker comprises determining S206C_1 one or more of: a motion parameter, a trembling parameter, a shaking parameter, and a tic parameter. In one or more example methods, the first biokinetic biomarker is based on one or more of: a motion parameter, a trembling parameter, a shaking parameter, and a tic parameter.

In one or more example methods, the electronic device comprises one or more sensors and the method comprises obtaining S204 internal sensor data from the one or more sensors. In one or more example methods, the health parameter is based on the internal sensor data.

In one or more example methods, the processor comprises machine learning circuitry configured to operate according to a machine learning model. In one or more example methods, determining S206 a health parameter comprises determining S206D the health parameter, based on the sensor data, using the machine learning model.

In one or more example methods, the interface comprises one or more of: a Bluetooth interface, Bluetooth low energy interface, and a magnetic induction interface.

Figs. 4A-4B show a flow diagram of an example method, such as a method 300.

A method 300 of operating a hearing system is disclosed. The method 300, may be performed by a system as disclosed herein, such as system 2.

The method 300 comprises obtaining S302 external sensor data from a hearing device. The method 300 comprises determining S306, based on the sensor data, a health parameter indicative of a cognitive state of a user of the hearing device. The method 300 comprises determining S308 whether the health parameter satisfies a first criterion indicative of a cognitive decline. The method 300 comprises, in accordance with the health parameter satisfying the first criterion, outputting S310 a health representation associated with the cognitive state.

Optionally, in accordance with the health parameter not satisfying the first criterion, the method 300 comprises refraining S309 from outputting S310 a health representation associated with the cognitive state.

In one or more example methods, outputting S310 the health representation comprises displaying S310A a user interface representing the health representation.

In one or more example methods, in accordance with the first criterion being satisfied, the method comprises performing S312 a cognitive test scheme.

In one or more example methods, the external sensor data comprises microphone input data. In one or more example methods, the health parameter is based on the microphone input data.

In one or more example methods, determining S306 a health parameter comprises determining S306A, based on the microphone input data, a first voice biomarker. In one or more example methods, the first criterion is based on the first voice biomarker.

In one or more example methods, determining S306A the first voice biomarker comprises determining S306A_1 one or more of: a linguistic parameter, an acoustic parameter, a verbal fluency parameter, a mumbling parameter, a voice pitch parameter, and a speech rhythm parameter. In one or more example methods, the first voice biomarker is based on one or more of: a linguistic parameter, an acoustic parameter, a verbal fluency parameter, a mumbling parameter, a voice pitch parameter, and a speech rhythm parameter.

In one or more example methods, the external sensor data comprises physiological data. In one or more example methods, the health parameter is based on the physiological data.

In one or more example methods, determining S306 a health parameter comprises determining 306B, based on the physiological data, a first physiological biomarker. In one or more example methods, the first criterion is based on the first physiological biomarker.

In one or more example methods, determining S306B a first physiological biomarker comprises determining S306B_1 one or more of: a blood pressure parameter, a blood flow parameter, a heart rate parameter, a respiratory parameter, a temperature parameter, an oxygen parameter, and a brain activity parameter. In one or more example methods, the first physiological biomarker is based on one or more of: the blood pressure parameter, the blood flow parameter, the heart rate parameter, the respiratory parameter, the temperature parameter, the oxygen parameter, and the brain activity parameter.

In one or more example methods, the external sensor data comprises biokinetic data. In one or more example methods, the health parameter is based on the biokinetic data.

In one or more example methods, determining S306 a health parameter comprises determining S306C, based on the biokinetic data, a first biokinetic biomarker. In one or more example methods, the first criterion is based on the first biokinetic biomarker.

In one or more example methods, determining S306C a first biokinetic biomarker comprises determining S306C_1 one or more of: a motion parameter, a trembling parameter, a shaking parameter, and a tic parameter. In one or more example methods, the first biokinetic biomarker is based on one or more of: a motion parameter, a trembling parameter, a shaking parameter, and a tic parameter.

In one or more example methods, the electronic device comprises one or more sensors and the method comprises obtaining S304 internal sensor data from the one or more sensors. In one or more example methods, the health parameter is based on the internal sensor data.

In one or more example methods, the electronic device comprises machine learning circuitry configured to operate according to a machine learning model. In one or more example methods, determining S306 a health parameter comprises determining S306D the health parameter, based on the external sensor data, using the machine learning model.

In one or more example methods, the interface of the electronic device and/or the interface of the hearing device comprises one or more of: a Bluetooth interface, Bluetooth low energy interface, and a magnetic induction interface.

In one or more example methods, the electronic device is a server device.

In one or more example methods, the method comprises training S314 the machine learning model, providing S316 an updated machine learning model and transmitting S318 to the electronic device, the updated machine learning model.

In one or more example methods, the electronic device is an accessory device.

Examples of electronic devices, systems, and methods according to the disclosure are set out in the following items:
A1. A hearing device, the hearing device comprising
   a memory;
   an interface;
   a processor; and
   one or more sensors;
   wherein the processor is configured to:
   obtain sensor data from the one or more sensors;
   determine, based on the sensor data, a health parameter indicative of a cognitive state of a user of the hearing device;
   determine whether the health parameter satisfies a first criterion indicative of a cognitive decline; and
   in accordance with the health parameter satisfying the first criterion, output, via the interface, a health representation associated with the cognitive state.
A2. Hearing device according to item A1, wherein the one or more sensors comprise a microphone for provision of microphone input data, and wherein the health parameter is based on the microphone input data.
A3. Hearing device according to item A2, wherein to determine a health parameter comprises to determine, based on the microphone input data, a first voice biomarker, and wherein the first criterion is based on the first voice biomarker.
A4. Hearing device according to item A3, wherein to determine the first voice biomarker comprises to determine one or more of: a linguistic parameter, an acoustic parameter, a verbal fluency parameter, a mumbling parameter, a voice pitch parameter, and a speech rhythm parameter; and wherein the first voice biomarker is based on one or more of: a linguistic parameter, an acoustic parameter, a verbal fluency parameter, a mumbling parameter, a voice pitch parameter, and a speech rhythm parameter.
A5. Hearing device according to any one of the previous items, wherein the one or more sensors comprise one or more physiological sensors for provision of physiological data, and wherein the health parameter is based on the physiological data.
A6. Hearing device according to item A5, wherein to determine a health parameter comprises to determine, based on the physiological data, a first physiological biomarker, and wherein the first criterion is based on the first physiological biomarker.
A7. Hearing device according to item A6, wherein to determine a first physiological biomarker comprises to determine one or more of: a blood pressure parameter, a blood flow parameter, a heart rate parameter, a respiratory parameter, a temperature parameter, an oxygen parameter, and a brain activity parameter; and wherein the first physiological biomarker is based on one or more of: the blood pressure parameter, the blood flow parameter, the heart rate parameter, the respiratory parameter, the temperature parameter, the oxygen parameter, and the brain activity parameter.
A8. Hearing device according to any one of the previous items, wherein the one or more sensors comprise one or more biokinetic sensors for provision of biokinetic data, and wherein the health parameter is based on the biokinetic data.
A9. Hearing device according to item A8, wherein to determine a health parameter comprises to determine, based on the biokinetic data, a first biokinetic biomarker, and wherein the first criterion is based on the first biokinetic biomarker.
A10. Hearing device according to item A9, wherein to determine a first biokinetic biomarker comprises to determine one or more of: a motion parameter, a trembling parameter, a shaking parameter, and a tic parameter; and wherein the first biokinetic biomarker is based on one or more of: a motion parameter, a trembling parameter, a shaking parameter, and a tic parameter.
A11. Hearing device according to any of the previous items, wherein the one or more sensors comprise one or more of: an optical sensor, a magnetic sensor, a galvanic sensor, a motion sensor, and a capacitive sensor.
A12. Hearing device according to any of the previous items, wherein the processor comprises machine learning circuitry configured to operate according to a machine learning model, wherein to determine a health parameter comprises to determine the health parameter, based on the sensor data, using the machine learning model.
A13. Method of operating a hearing device, the method comprising:
   obtaining, from one or more sensors of the hearing device, sensor data;
   determining, based on the sensor data, a health parameter indicative of a cognitive state of a user of the hearing device;
   determining whether the health parameter satisfies a first criterion indicative of a cognitive decline; and
   in accordance with the health parameter satisfying the first criterion, outputting a health representation associated with the cognitive state.
A14. The method of operating a hearing device according to item A13, wherein the one or more sensors comprise a microphone for provision of microphone input data, and wherein the health parameter is based on the microphone input data.
A15. The method of operating a hearing device according to item A14, wherein determining (S104) a health parameter comprises:
   determining (S104A), based on the microphone input data, a first voice biomarker, and wherein the first criterion is based on the first voice biomarker.
A16. The method of operating a hearing device according to item A15, wherein determining (S104A) the first voice biomarker comprises:
   determining (S104A_1) one or more of: a linguistic parameter, an acoustic parameter, a verbal fluency parameter, a mumbling parameter, a voice pitch parameter, and a speech rhythm parameter; and wherein the first voice biomarker is based on one or more of: a linguistic parameter, an acoustic parameter, a verbal fluency parameter, a mumbling parameter, a voice pitch parameter, and a speech rhythm parameter.
A17. The method of operating a hearing device according to any of items A13-A16, wherein the one or more sensors comprise one or more physiological sensors for provision of physiological data, and wherein the health parameter is based on the physiological data.
A18. The method of operating a hearing device according to item A17, wherein determining (S104) a health parameter comprises:
   determining (S104B), based on the physiological data, a first physiological biomarker, and wherein the first criterion is based on the first physiological biomarker.
A19. The method of operating a hearing device according to item A18, wherein determining (S104B) a first physiological biomarker comprises:
   determining (S104B_1) one or more of: a blood pressure parameter, a blood flow parameter, a heart rate parameter, a respiratory parameter, a temperature parameter, an oxygen parameter, and a brain activity parameter; and wherein the first physiological biomarker is based on one or more of: the blood pressure parameter, the blood flow parameter, the heart rate parameter, the respiratory parameter, the temperature parameter, the oxygen parameter, and the brain activity parameter.
A20. The method of operating a hearing device according to any of items A13-A19, wherein the one or more sensors comprise one or more biokinetic sensors for provision of biokinetic data, and wherein the health parameter is based on the biokinetic data.
A21. The method of operating a hearing device according to item A20, wherein determining (S104) a health parameter comprises:
   determining (S104C), based on the biokinetic data, a first biokinetic biomarker, and wherein the first criterion is based on the first biokinetic biomarker.
A22. The method of operating a hearing device according to item A21, wherein determining (S104C) a first biokinetic biomarker comprises:
   determining (S104C_1) one or more of: a motion parameter, a trembling parameter, a shaking parameter, and a tic parameter; and wherein the first biokinetic biomarker is based on one or more of: a motion parameter, a trembling parameter, a shaking parameter, and a tic parameter.
A23. The method of operating a hearing device according to any of items A13-A22, wherein the one or more sensors comprise one or more of: an optical sensor, a magnetic sensor, a galvanic sensor, a motion sensor, and a capacitive sensor.
A24. The method of operating a hearing device according to any of items A13-A23, wherein the processor comprises machine learning circuitry configured to operate according to a machine learning model, wherein determining (S104) a health parameter comprises:
   determining (S104D) the health parameter, based on the sensor data, using the machine learning model.
B1. An electronic device, the electronic device comprising
   a memory;
   an interface; and
   a processor;
   wherein the processor is configured to:
   obtain, via the interface, external sensor data from a hearing device;
   determine, based on the external sensor data, a health parameter indicative of a cognitive state of a user of the hearing device;
   determine whether the health parameter satisfies a first criterion indicative of a cognitive decline; and
   in accordance with the health parameter satisfying the first criterion, output, via the interface, a health representation associated with the cognitive state.
B2. Electronic device according to item B1, wherein to output the health representation comprises to display, via the interface, a user interface representing the health representation.
B3. Electronic device according to any of items B1-B2, wherein in accordance with the first criterion being satisfied, the electronic device is configured to perform a cognitive test scheme.
B4. Electronic device according to any of items B1-B3, wherein the external sensor data comprises microphone input data, and wherein the health parameter is based on the microphone input data.
B5. Electronic device according to item B4, wherein to determine a health parameter comprises to determine, based on the microphone input data, a first voice biomarker, and wherein the first criterion is based on the first voice biomarker.
B6. Electronic device according to item B5, wherein to determine the first voice biomarker comprises to determine one or more of: a linguistic parameter, an acoustic parameter, a verbal fluency parameter, a mumbling parameter, a voice pitch parameter, and a speech rhythm parameter; and wherein the first voice biomarker is based on one or more of: a linguistic parameter, an acoustic parameter, a verbal fluency parameter, a mumbling parameter, a voice pitch parameter, and a speech rhythm parameter.
B7. Electronic device according to any of items B1-B6, wherein the external sensor data comprises physiological data, and wherein the health parameter is based on the physiological data.
B8. Electronic device according to item B7, wherein to determine a health parameter comprises to determine, based on the physiological data, a first physiological biomarker, and wherein the first criterion is based on the first physiological biomarker.
B9. Electronic device according to item B8, wherein to determine a first physiological biomarker comprises to determine one or more of: a blood pressure parameter, a blood flow parameter, a heart rate parameter, a respiratory parameter, a temperature parameter, an oxygen parameter, and a brain activity parameter; and wherein the first physiological biomarker is based on one or more of: the blood pressure parameter, the blood flow parameter, the heart rate parameter, the respiratory parameter, the temperature parameter, the oxygen parameter, and the brain activity parameter.
B10. Electronic device according to any of items B1-B9, wherein the external sensor data comprises biokinetic data, and wherein the health parameter is based on the biokinetic data.
B11. Electronic device according to item B10, wherein to determine a health parameter comprises to determine, based on the biokinetic data, a first biokinetic biomarker, and wherein the first criterion is based on the first biokinetic biomarker.
B12. Electronic device according to item B11, wherein to determine a first biokinetic biomarker comprises to determine one or more of: a motion parameter, a trembling parameter, a shaking parameter, and a tic parameter; and wherein the first biokinetic biomarker is based on one or more of: a motion parameter, a trembling parameter, a shaking parameter, and a tic parameter.
B13. Electronic device according to any of items B1-B12, wherein the electronic device comprises one or more sensors and wherein the processor is configured to obtain internal sensor data from the one or more sensors, and wherein the health parameter is based on the internal sensor data.
B14. Electronic device according to any of items B1-B13, wherein the processor comprises machine learning circuitry configured to operate according to a machine learning model, wherein to determine a health parameter comprises to determine the health parameter, based on the external sensor data, using the machine learning model.
B15. Electronic device according to any of items B1-B14, wherein the interface comprises one or more of: a Bluetooth interface, Bluetooth low energy interface, and a magnetic induction interface.
B16. Method of operating an electronic device, the method comprising:
   obtaining external sensor data from a hearing device;
   determining, based on the external sensor data, a health parameter indicative of a cognitive state of a user of the hearing device;
   determining whether the health parameter satisfies a first criterion indicative of a cognitive decline; and
   in accordance with the health parameter satisfying the first criterion, outputting a health representation associated with the cognitive state.
B17. The method of operating an electronic device according to item B16, wherein outputting (S110) the health representation comprises:
   displaying (S110A) a user interface representing the health representation.
B18. The method of operating an electronic device according to any of items B16-B17, wherein in accordance with the first criterion being satisfied, the method comprises:
   performing (S112) a cognitive test scheme.
B19. The method of operating an electronic device according to any of items B16-B18, wherein the external sensor data comprises microphone input data, and wherein the health parameter is based on the microphone input data.
B20. The method of operating an electronic device according to item B19, wherein determining (S106) a health parameter comprises:
   determining (S106A), based on the microphone input data, a first voice biomarker, and wherein the first criterion is based on the first voice biomarker.
B21. The method of operating an electronic device according to item B20, wherein determining (S106A) the first voice biomarker comprises:
   determining (S106A_1) one or more of: a linguistic parameter, an acoustic parameter, a verbal fluency parameter, a mumbling parameter, a voice pitch parameter, and a speech rhythm parameter; and wherein the first voice biomarker is based on one or more of: a linguistic parameter, an acoustic parameter, a verbal fluency parameter, a mumbling parameter, a voice pitch parameter, and a speech rhythm parameter.
B22. The method of operating an electronic device according to any of items B16-B21, wherein the external sensor data comprises physiological data, and wherein the health parameter is based on the physiological data.
B23. The method of operating an electronic device according to item B22, wherein determining (S106) a health parameter comprises:
   determining (106B), based on the physiological data, a first physiological biomarker, and wherein the first criterion is based on the first physiological biomarker.
B24. The method of operating an electronic device according to item B23, wherein determining (S106B) a first physiological biomarker comprises:
   determining (S106B_1) one or more of: a blood pressure parameter, a blood flow parameter, a heart rate parameter, a respiratory parameter, a temperature parameter, an oxygen parameter, and a brain activity parameter; and wherein the first physiological biomarker is based on one or more of: the blood pressure parameter, the blood flow parameter, the heart rate parameter, the respiratory parameter, the temperature parameter, the oxygen parameter, and the brain activity parameter.
B25. The method of operating an electronic device according to any of items B16-B24, wherein the external sensor data comprises biokinetic data, and wherein the health parameter is based on the biokinetic data.
B26. The method of operating an electronic device according to item B25, wherein determining (S106) a health parameter comprises:
   determining (S106C), based on the biokinetic data, a first biokinetic biomarker, and wherein the first criterion is based on the first biokinetic biomarker.
B27. The method of operating an electronic device according to item B26, wherein determining (S106C) a first biokinetic biomarker comprises:
   determining (S106C_1) one or more of: a motion parameter, a trembling parameter, a shaking parameter, and a tic parameter; and wherein the first biokinetic biomarker is based on one or more of: a motion parameter, a trembling parameter, a shaking parameter, and a tic parameter.
B28. The method of operating an electronic device according to any of items B16-B27, wherein the electronic device comprises one or more sensors and wherein the method comprises:
   obtaining (S104) internal sensor data from the one or more sensors, and wherein the health parameter is based on the internal sensor data.
B29. The method of operating an electronic device according to any of items B16-B28, wherein the processor comprises machine learning circuitry configured to operate according to a machine learning model, wherein determining (S106) a health parameter comprises:
   determining (S106D) the health parameter, based on the sensor data, using the machine learning model.
B30. The method of operating an electronic device according to any of items B16-B29, wherein the interface comprises one or more of: a Bluetooth interface, Bluetooth low energy interface, and a magnetic induction interface.
C1. A hearing system, the system comprising
   an electronic device comprising a memory, an interface, and a processor; and
   a hearing device comprising a memory, an interface, a processor, and one or more sensors;
   wherein the system is configured to:
   obtain external sensor data from the one or more sensors;
   determine, based on the external sensor data, a health parameter indicative of a cognitive state of a user of the hearing device;
   determine whether the health parameter satisfies a first criterion indicative of a cognitive decline; and
   in accordance with the health parameter satisfying the first criterion, output a health representation associated with the cognitive state.
C2. Hearing system according to item C1, wherein to output the health representation comprises to display, via the interface of the electronic device, a user interface representing the health representation.
C3. Hearing system according to any of items C1-C2, wherein in accordance with the first criterion being satisfied, the system is configured to perform, via the interface of the electronic device, a cognitive test scheme.
C4. Hearing system according to any of items C1-C3, wherein the external sensor data comprises microphone input data, and wherein the health parameter is based on the microphone input data.
C5. Hearing system according to item C4, wherein to determine a health parameter comprises to determine, based on the microphone input data, a first voice biomarker, and wherein the first criterion is based on the first voice biomarker.
C6. Hearing system according to item C5, wherein to determine the first voice biomarker comprises to determine one or more of: a linguistic parameter, an acoustic parameter, a verbal fluency parameter, a mumbling parameter, a voice pitch parameter, and a speech rhythm parameter; and wherein the first voice biomarker is based on one or more of: a linguistic parameter, an acoustic parameter, a verbal fluency parameter, a mumbling parameter, a voice pitch parameter, and a speech rhythm parameter.
C7. Hearing system according to any of items C1-C6, wherein the external sensor data comprises physiological data, and wherein the health parameter is based on the physiological data.
C8. Hearing system according to item C7, wherein to determine a health parameter comprises to determine, based on the physiological data, a first physiological biomarker, and wherein the first criterion is based on the first physiological biomarker.
C9. Hearing system according to item C8, wherein to determine a first physiological biomarker comprises to determine one or more of: a blood pressure parameter, a blood flow parameter, a heart rate parameter, a respiratory parameter, a temperature parameter, an oxygen parameter, and a brain activity parameter; and wherein the first physiological biomarker is based on one or more of: the blood pressure parameter, the blood flow parameter, the heart rate parameter, the respiratory parameter, the temperature parameter, the oxygen parameter, and the brain activity parameter.
C10. Hearing system according to any of items C1-C9, wherein the external sensor data comprises biokinetic data, and wherein the health parameter is based on the biokinetic data.
C11. Hearing system according to item C10, wherein to determine a health parameter comprises to determine, based on the biokinetic data, a first biokinetic biomarker, and wherein the first criterion is based on the first biokinetic biomarker.
C12. Hearing system according to item C11, wherein to determine a first biokinetic biomarker comprises to determine one or more of: a motion parameter, a trembling parameter, a shaking parameter, and a tic parameter; and wherein the first biokinetic biomarker is based on one or more of: a motion parameter, a trembling parameter, a shaking parameter, and a tic parameter.
C13. Hearing system according to any of items C1-C12, wherein the electronic device comprises one or more sensors and wherein the system is configured to obtain internal sensor data from the one or more sensors of the electronic device, and wherein the health parameter is based on the internal sensor data.
C14. Hearing system according to any of items C1-C13, wherein the hearing system comprises machine learning circuitry configured to operate according to a machine learning model, wherein to determine a health parameter comprises to determine the health parameter, based on the external sensor data, using the machine learning model.
C15. Hearing system according to any of items C1-C14, wherein the interface of the electronic device and/or the interface of the hearing device comprises one or more of: a Bluetooth interface, Bluetooth low energy interface, and a magnetic induction interface.
C16. Hearing system according to any of items C1-C15, wherein the electronic device is a server device.
C17. Hearing system according to item C16, wherein the server device is configured to train the machine learning model, to provide an updated machine learning model, and to transmit, to the electronic device and/or the hearing device, the updated machine learning model.
C18. Hearing system according to any of items C1-C17, wherein the electronic device is an accessory device.
C19. Method of operating a hearing system, the method comprising:
   obtaining external sensor data from a hearing device;
   determining, based on the external sensor data, a health parameter indicative of a cognitive state of a user of the hearing device;
   determining whether the health parameter satisfies a first criterion indicative of a cognitive decline; and
   in accordance with the health parameter satisfying the first criterion, outputting a health representation associated with the cognitive state.
C20. The method of operating a hearing system according to item C19, wherein outputting (S110) the health representation comprises:
   displaying (S110A) a user interface representing the health representation.
C21. The method of operating a hearing system according to any of items C19-C20, wherein in accordance with the first criterion being satisfied, the method comprises:
   performing (S112) a cognitive test scheme.
C22. The method of operating a hearing system according to any of items C19-C22, wherein the external sensor data comprises microphone input data, and wherein the health parameter is based on the microphone input data.
C23. The method of operating a hearing system according to item C22, wherein determining (S106) a health parameter comprises:
   determining (S106A), based on the microphone input data, a first voice biomarker, and wherein the first criterion is based on the first voice biomarker.
C24. The method of operating a hearing system according to item C23, wherein determining (S106A) the first voice biomarker comprises:
   determining (S106A_1) one or more of: a linguistic parameter, an acoustic parameter, a verbal fluency parameter, a mumbling parameter, a voice pitch parameter, and a speech rhythm parameter; and wherein the first voice biomarker is based on one or more of: a linguistic parameter, an acoustic parameter, a verbal fluency parameter, a mumbling parameter, a voice pitch parameter, and a speech rhythm parameter.
C25. The method of operating a hearing system according to any of items C19-C24, wherein the external sensor data comprises physiological data, and wherein the health parameter is based on the physiological data.
C26. The method of operating a hearing system according to item C25, wherein determining (S106) a health parameter comprises:
   determining (106B), based on the physiological data, a first physiological biomarker, and wherein the first criterion is based on the first physiological biomarker.
C27. The method of operating a hearing system according to item C26, wherein determining (S106B) a first physiological biomarker comprises:
   determining (S106B_1) one or more of: a blood pressure parameter, a blood flow parameter, a heart rate parameter, a respiratory parameter, a temperature parameter, an oxygen parameter, and a brain activity parameter; and wherein the first physiological biomarker is based on one or more of: the blood pressure parameter, the blood flow parameter, the heart rate parameter, the respiratory parameter, the temperature parameter, the oxygen parameter, and the brain activity parameter.
C28. The method of operating a hearing system according to any of items C19-C27, wherein the external sensor data comprises biokinetic data, and wherein the health parameter is based on the biokinetic data.
C29. The method of operating a hearing system according to item C28, wherein determining (S106) a health parameter comprises:
   determining (S106C), based on the biokinetic data, a first biokinetic biomarker, and wherein the first criterion is based on the first biokinetic biomarker.
C30. The method of operating a hearing system according to item C29, wherein determining (S106C) a first biokinetic biomarker comprises:
   determining (S106C_1) one or more of: a motion parameter, a trembling parameter, a shaking parameter, and a tic parameter; and wherein the first biokinetic biomarker is based on one or more of: a motion parameter, a trembling parameter, a shaking parameter, and a tic parameter.
C31. The method of operating a hearing system according to any of items C19-C30, wherein the electronic device comprises one or more sensors and wherein the method comprises:
   obtaining (S104) internal sensor data from the one or more sensors, and wherein the health parameter is based on the internal sensor data.
C32. The method of operating a hearing system according to any of items C19-C31, wherein the electronic device comprises machine learning circuitry configured to operate according to a machine learning model, wherein determining (S106) a health parameter comprises:
   determining (S106D) the health parameter, based on the external sensor data, using the machine learning model.
C33. The method of operating a hearing system according to any of items C19-C32, wherein the interface of the electronic device and/or the interface of the hearing device comprises one or more of: a Bluetooth interface, Bluetooth low energy interface, and a magnetic induction interface.
C34. The method of operating a hearing system according to any of items C19-C33, wherein the electronic device is a server device.
C35. The method of operating a hearing system according to item C34, wherein the method comprises:
   training (S114) the machine learning model;
   providing (S116) an updated machine learning model; and
   transmitting (S118), to the electronic device, the updated machine learning model.
C36. The method of operating a hearing system according to any of items C19-C35, wherein the electronic device is an accessory device.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

It may be appreciated that the Figures comprise some circuitries or operations which are illustrated with a solid line and some circuitries, components, features, or operations which are illustrated with a dashed line. Circuitries or operations which are comprised in a solid line are circuitries, components, features or operations which are comprised in the broadest example. Circuitries, components, features, or operations which are comprised in a dashed line are examples which may be comprised in, or a part of, or are further circuitries, components, features, or operations which may be taken in addition to circuitries, components, features, or operations of the solid line examples. It should be appreciated that these operations need not be performed in order presented. Furthermore, it should be appreciated that not all of the operations need to be performed. The example operations may be performed in any order and in any combination. It should be appreciated that these operations need not be performed in order presented. Circuitries, components, features, or operations which are comprised in a dashed line may be considered optional.

Other operations that are not described herein can be incorporated in the example operations. For example, one or more additional operations can be performed before, after, simultaneously, or between any of the described operations.

Certain features discussed above as separate implementations can also be implemented in combination as a single implementation. Conversely, features described as a single implementation can also be implemented in multiple implementations separately or in any suitable sub-combination. Moreover, although features may be described above as acting in certain combinations, one or more features from a claimed combination can, in some cases, be excised from the combination, and the combination may be claimed as any sub-combination or variation of any sub-combination.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the examples may be implemented at least in part by means of both hardware and software, and that several "means", "units" or "devices" may be represented by the same item of hardware.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than or equal to 10% of, within less than or equal to 5% of, within less than or equal to 1% of, within less than or equal to 0.1% of, and within less than or equal to 0.01% of the stated amount. If the stated amount is 0 (e.g., none, having no), the above recited ranges can be specific ranges, and not within a particular % of the value. For example, within less than or equal to 10 wt./vol. % of, within less than or equal to 5 wt./vol. % of, within less than or equal to 1 wt./vol. % of, within less than or equal to 0.1 wt./vol. % of, and within less than or equal to 0.01 wt./vol. % of the stated amount.

Although features have been shown and described, it will be understood that they are not intended to limit the claimed disclosure, and it will be made obvious to those skilled in the art that various changes and modifications may be made without departing from the scope of the claimed disclosure. The specification and drawings are, accordingly, to be regarded in an illustrative rather than restrictive sense. The claimed disclosure is intended to cover all alternatives, modifications, and equivalents.

### LIST OF REFERENCES

- 1, 1A, 1B: user
- 2: system
- 4: input, user input
- 6: health representation, output
- 10: electronic device, accessory device
- 10A: one or more interfaces
- 10B: memory
- 10C: processor
- 10D: one or more sensors
- 12: machine learning circuitry
- 13: control, transmit
- 14: obtain
- 16: obtain
- 18: control, transmit
- 20: electronic device, server device
- 20A: memory
- 20B: one or more interfaces
- 20C: processor(s)
- 21: machine learning circuitry
- 22: control, transmit
- 24: obtain
- 30: hearing device
- 30A: one or more interfaces
- 30B: memory
- 30C: processor
- 30D: one or more sensors
- 31: machine learning circuitry
- 32: control, transmit
- 34: obtain
- 36: health representation, output
- 38: input, user input
- 40: network

## Claims

1. Method of operating a hearing system, the method comprising:
obtaining external sensor data from a hearing device;
determining, based on the external sensor data, a health parameter indicative of a cognitive state of a user of the hearing device;
determining whether the health parameter satisfies a first criterion indicative of a cognitive decline; and
in accordance with the health parameter satisfying the first criterion, outputting a health representation associated with the cognitive state.

2. The method of operating a hearing system according to claim 1, wherein outputting (S110) the health representation comprises:
displaying (S110A) a user interface representing the health representation.

3. The method of operating a hearing system according to any of claims 1-2, wherein in accordance with the first criterion being satisfied, the method comprises:
performing (S112) a cognitive test scheme.

4. The method of operating a hearing system according to any of claims 1-3, wherein the external sensor data comprises microphone input data, and wherein the health parameter is based on the microphone input data, wherein determining (S106) a health parameter comprises:
determining (S106A), based on the microphone input data, a first voice biomarker, and wherein the first criterion is based on the first voice biomarker.

5. The method of operating a hearing system according to claim 4, wherein determining (S106A) the first voice biomarker comprises:
determining (S106A_1) one or more of: a linguistic parameter, an acoustic parameter, a verbal fluency parameter, a mumbling parameter, a voice pitch parameter, and a speech rhythm parameter; and wherein the first voice biomarker is based on one or more of: a linguistic parameter, an acoustic parameter, a verbal fluency parameter, a mumbling parameter, a voice pitch parameter, and a speech rhythm parameter.

6. The method of operating a hearing system according to any of the preceding claims, wherein the external sensor data comprises physiological data, and wherein the health parameter is based on the physiological data.

7. The method of operating a hearing system according to claim 6, wherein determining (S106) a health parameter comprises:
determining (106B), based on the physiological data, a first physiological biomarker, and wherein the first criterion is based on the first physiological biomarker.

8. The method of operating a hearing system according to claim 7, wherein determining (S106B) a first physiological biomarker comprises:
determining (S106B_1) one or more of: a blood pressure parameter, a blood flow parameter, a heart rate parameter, a respiratory parameter, a temperature parameter, an oxygen parameter, and a brain activity parameter; and wherein the first physiological biomarker is based on one or more of: the blood pressure parameter, the blood flow parameter, the heart rate parameter, the respiratory parameter, the temperature parameter, the oxygen parameter, and the brain activity parameter.

9. The method of operating a hearing system according to any of the preceding claims, wherein the external sensor data comprises biokinetic data, and wherein the health parameter is based on the biokinetic data.

10. The method of operating a hearing system according to claim 9, wherein determining (S106) a health parameter comprises:
determining (S106C), based on the biokinetic data, a first biokinetic biomarker, and wherein the first criterion is based on the first biokinetic biomarker.

11. The method of operating a hearing system according to claim 10, wherein determining (S106C) a first biokinetic biomarker comprises:
determining (S106C_1) one or more of: a motion parameter, a trembling parameter, a shaking parameter, and a tic parameter; and wherein the first biokinetic biomarker is based on one or more of: a motion parameter, a trembling parameter, a shaking parameter, and a tic parameter.

12. The method of operating a hearing system according to any of the preceding claims, wherein the electronic device comprises one or more sensors and wherein the method comprises:
obtaining (S104) internal sensor data from the one or more sensors, and wherein the health parameter is based on the internal sensor data.

13. The method of operating a hearing system according to any of the preceding claims, wherein the electronic device comprises machine learning circuitry configured to operate according to a machine learning model, wherein determining (S106) a health parameter comprises:
determining (S106D) the health parameter, based on the external sensor data, using the machine learning model.

14. The method of operating a hearing system according to claim 13, wherein the method comprises:
training (S114) the machine learning model;
providing (S116) an updated machine learning model; and
transmitting (S118), to the electronic device, the updated machine learning model.

15. A hearing system, the system comprising
an electronic device comprising a memory, an interface, and a processor; and
a hearing device comprising a memory, an interface, a processor, and one or more sensors;
wherein the system is configured to:
obtain external sensor data from the one or more sensors;
determine, based on the external sensor data, a health parameter indicative of a cognitive state of a user of the hearing device;
determine whether the health parameter satisfies a first criterion indicative of a cognitive decline; and
in accordance with the health parameter satisfying the first criterion, output a health representation associated with the cognitive state.
